# EUROPEAN PATENT SPECIFICATION

(11) **EP 3 194 027 B1**
(45) Date of publication and mention of the grant of the patent: **11.02.2026**
(21) Application number: 15842621.3
(22) Date of filing: 15.09.2015
(51) Int. Cl.: A61K 31/12, A61K 31/198, A61K 31/41, A61K 31/4515, A61K 31/519, A61K 31/5415, A61K 31/551, A61P 25/14, A61K 45/06, A61P 25/18, A61P 25/24, A61P 43/00, A61P 39/06, A61P 25/22

(54) **COMPOSITIONS FOR USE IN TREATING PSYCHOTIC DISORDERS**
ZUSAMMENSETZUNGEN ZUR BEHANDLUNG VON PSYCHOTISCHER ERKRANKUNGEN
COMPOSITIONS POUR LE TRAITEMENT DE TROUBLES PSYCHOTIQUES

(30) Priority: 15.09.2014 US 201462050635 P
(43) Date of publication of application: 26.07.2017
(73) Proprietor: Sound Pharmaceuticals Incorporated, Seattle, WA 98103 (US)
(72) Inventor: KIL, Jonathan, Seattle, WA 98103 (US)
(74) Representative: Elkington and Fife LLP
(86) International application number: PCT/US2015/050255
(87) International publication number: WO 2016/044314

(56) References cited:
- WO-A1-03/026684
- WO-A1-2013/016727
- WO-A1-98/29101
- US-A1- 2010 099 762
- US-A1- 2010 137 441
- US-A1- 2011 046 090
- BURGER M E ET AL: "Ebselen attenuates haloperidol-induced orofacial dyskinesia and oxidative stress in rat brain", PHARMACOLOGY BIOCHEMISTRY AND BEHAVIOR, ELSEVIER, US, vol. 81, no. 3, 1 July 2005 (2005-07-01), pages 608 - 615, XP027691061, ISSN: 0091-3057, [retrieved on 20050701]
- MARILISE E. BURGER ET AL: "Ebselen attenuates reserpine-induced orofacial dyskinesia and oxidative stress in rat striatum", PROGRESS IN NEURO-PSYCHOPHARMACOLOGY & BIOLOGICAL PSYCHIATRY, vol. 27, no. 1, 18 November 2002 (2002-11-18), GB, pages 135 - 140, XP055457469, ISSN: 0278-5846, DOI: 10.1016/S0278-5846(02)00344-5
- AHMAD GHANIZADEH ET AL: "A randomized double blind placebo controlled clinical trial of N-Acetylcysteine added to risperidone for treating autistic disorders", BMC PSYCHIATRY, BIOMED CENTRAL, LONDON, GB, vol. 13, no. 1, 25 July 2013 (2013-07-25), pages 196, XP021157847, ISSN: 1471-244X, DOI: 10.1186/1471-244X-13-196
- JAN-HARRY CABUNGCAL ET AL: "Juvenile Antioxidant Treatment Prevents Adult Deficits in a Developmental Model of Schizophrenia", NEURON, vol. 83, no. 5, 14 September 2014 (2014-09-14), US, pages 1073 - 1084, XP055699640, ISSN: 0896-6273, DOI: 10.1016/j.neuron.2014.07.028
- SHARPLEY ANN L ET AL: "A phase 2a randomised, double-blind, placebo-controlled, parallel-group, add-on clinical trial of ebselen (SPI-1005) as a novel treatment for mania or hypomania", PSYCHOPHARMACOLOGY, vol. 237, no. 12, 9 September 2020 (2020-09-09), pages 3773 - 3782, XP037302338, ISSN: 0033-3158, DOI: 10.1007/S00213-020-05654-1
- MAGALH�ES PEDRO ET AL: "Antioxidant treatments for schizophrenia", COCHRANE LIBRARY, 5 February 2016 (2016-02-05), Chichester, UK, pages CD008919 - CD008919, XP055899380, [retrieved on 20220309], DOI: 10.1002/14651858.CD008919.pub2
- ALBERS ET AL.: "Low-dose fluvoxamine as an adjunct to reduce olanzapine therapeutic dose requirements: a prospective dose-adjusted drug interaction strategy.", J CLIN PSYCHOPHARMACOL., vol. 25, no. 2, April 2005 (2005-04-01), pages 170 - 4, XP009501093, Retrieved from the Internet <URL:http://www.ncbi.nlm.nih.gov/pubmed/15738749> [retrieved on 20151019]
- BEHR ET AL.: "Preclinical and Clinical Evidence of Antioxidant Effects of Antidepressant Agents: Implications for the Pathophysiology of Major Depressive Disorder.", OXIDATIVE MEDICINE AND CELLULAR LONGEVITY, vol. 2012, 2012, pages 1 - 13, XP055419380, Retrieved from the Internet <URL:http://downloads.hindawi.com/journals/oximed/2012/609421.pdf>

## Description

### CROSS REFERENCE TO RELATED APPLICATIONS

This application claims the benefit of U.S. Provisional Application No. 62/050,635, filed September 15, 2014.

### BACKGROUND

### Field of the invention

The invention relates to compositions and methods of treating GPx mediated disorders. In particular, the compositions comprise a combination of a glutathione peroxidase modulator and an antipsychotic agent. The references in the description to methods of treatment refer to the compounds, pharmaceutical compositions and medicaments of the present invention for use in a method for treatment of the human (or animal) body by therapy (or for diagnosis).

### Description of Related Art

For decades, dopamine was central to pathophysiological views and therapeutics for schizophrenia (SZ), followed by GABA and glutamate neurotransmission theories. More recently, the pathophysiology of SZ has been strongly linked to oxidative stress (Do et al., 2009; Kano et al., 2013). In active cells such as neurons of the central nervous system, natural antioxidative defenses include sequestration of free radicals by glutathione, through conversion of its reduced form, i.e. monomeric glutathione (GSH), by glutathione peroxidase (GPx) to its oxidized form, i.e. glutathione disulfide (GSSG). The primary function of GPx and the GSH→GPx→GSSG direction of the redox mechanism are to reduce free radicals, such as hydrogen peroxide (H₂O₂), peroxynitrite (ONOO⁻), and lipid hydroperoxides (LOOH) to their corresponding redox-inert counterparts, e.g. water and alcohols, and protect cell membranes, proteins and other structures from oxidative damage.

Many substrates and enzymes in the antioxidative system have been investigated in SZ, including GPx mediated changes. Studies showed changes in GPx during intial episodes of psychosis, when compensatory mechanisms are still able to combat oxidative stress. Overall, a strong correlation was shown to exist between GPx activity and SZ, although no drug currently exists that directly targets the GPx redox mechanism.

The present invention addresses these and other shortcomings of the prior art, as described below.

### SUMMARY

The present invention is set out in the appended claims.

In its many embodiments, the present disclosure provides novel combinations of compounds useful as, for example, glutathione peroxidase (GPx) modulators, methods of preparing such combinations, pharmaceutical compositions comprising one or more of such combinations, methods of preparing pharmaceutical compositions comprising one or more such combinations, and methods of treatment, prevention, inhibition or amelioration of one or more diseases associated with GPx mediated disorders using such combination or pharmaceutical compositions.

Some embodiments of a novel combination comprise at least two compounds or pharmaceutically acceptable salts thereof, wherein the first compound is a glutathione peroxidase mimic compound, and the second compound is an antipsychotic agent.

In some embodiments, the glutathione peroxidase modulator compound is selected from the group consisting of glutathione peroxidase mimic compounds, glutathione (GSH), glutathione prodrugs, and cysteine prodrugs.

In some embodiments, the antipsychotic agent is selected from the group consisting, Chlorpromazine (Thorazine), Haloperidol (Haldol), Perphenazine, Fluphenazine, Risperidone (Risperdal), Olanzapine (Zyprexa), Quetiapine (Seroquel), Ziprasidone (Geodon), Aripiprazole (Abilify), Paliperidone (Invega), Lurasidone (Latuda), and combinations thereof.

In some embodiments, a representative compound of a gluthione peroxidase mimic compound comprises ebselen, (2-phenyl-1,2-benzisoselenazol-3(2H)-one) with an empirical formula C₁₃H₉NOSe, molecular weight 274.2 and a formula of:

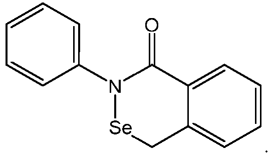

In some embodiments, gluthione peroxidase mimics comprise 2,2'-diseleno-bis-β-cyclodextrin and 6A,6B-diseleninic acid-6A',6B'-selenium bridged β-cyclodextrin.

In some embodiments, representative glutathione produgs comprise compounds of the formula: 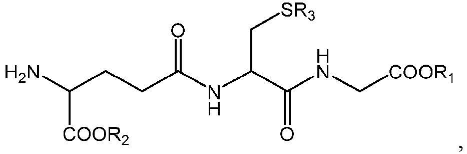 wherein
R₁ is H, methyl, ethyl, or isopropyl;
R₂ is H, or ethyl; and
R₃ is H, acetyl, phenylacetyl,

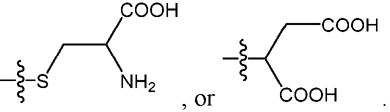

In some embodiments, a representative cysteine prodrug comprises N-acetyl cysteine (NAC) with a formula of:

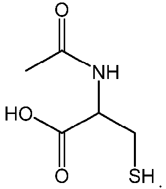

Some embodiments of cysteine prodrugs comprise N,N'-diacetyl-cysteine, N-acetyl cysteine amide, NAC esters (alkyl esters, glycolamide esters and acycloxymethyl esters), S-allyl cysteine, S-methyl cysteine, S-ethyl cysteine, S-propyl cysteine, or compounds of the formula: 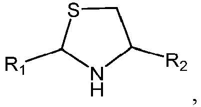 wherein
R₁ is H, oxo, methyl, ethyl, n-propyl, n-pentyl, phenyl, -(CHOH)ₙCH₂OH and wherein n is 1-5, or 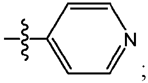 and
R₂ is H or -COOH.

Some embodiments of cysteine prodrugs comprise 2-substituted thiazolidine-4-carboxylic acids with aldose monosaccarides, such as glyceraldehyde, arabinose, lyxose, ribose, xylose, galactose, glucose, and mannose.

Another aspect of the present disclosure features a pharmaceutical composition comprising a novel combination and at least one pharmaceutically acceptable carrier.

Yet another aspect of the present disclose features a method of treating a subject suffering from or diagnosed with a disease, disorder, or condition mediated by GPx activity, comprising administering to the subject a therapeutically effective amount of a novel combination. Such disease, disorder, or condition can include, but is not limited to heightened oxidative stress, schizophrenia, bi-polar disorder, depression, mania, anxiety disorders or related psychotic disorders, tardive dyskinesia, and associated symptoms or complications thereof. The therapeutically effective amount of each compound included in the novel combination can be from about 0.1 mg/day to about 5000 mg/day, respectively.

Another aspect of the present disclosure features a method for reducing the side effects of administering an antipsychotic agent by co-administering a gluthione peroxidase modulator compound with the antipsychotic agent. In particular, reducing the side effects of administering an antipsychotic agent by co-administering ebselen with the antipsychotic agent. Even more particular, reducing the side effects of administering Chlorpromazine (Thorazine), Haloperidol (Haldol), Perphenazine, Fluphenazine, Risperidone (Risperdal), Olanzapine (Zyprexa), Quetiapine (Seroquel), Ziprasidone (Geodon), Aripiprazole (Abilify), Paliperidone (Invega), Lurasidone (Latuda) or combinations thereof by co-administering ebselen. In some embodiments, the side effects of administering an antipsychotic agent comprise tardive dyskinesia and other complications of administering an antipsychotic agent to a patient at high doses or over a long period of time.

The present disclosure further features a process for making a pharmaceutical composition comprising admixing any of the compounds of the novel combination and a pharmaceutically acceptable carrier.

Additional embodiments and their advantages will become apparent from the detailed discussion, schemes, examples, and claims below.

### BRIEF DESCRIPTION OF THE SEVERAL VIEWS OF THE DRAWINGS

These and other features, aspects, and advantages of the present invention will become better understood with regard to the following description, and accompanying drawings, where:

These and other features, aspects, and advantages of the present invention will become better understood with regard to the following description, and accompanying drawings, where:

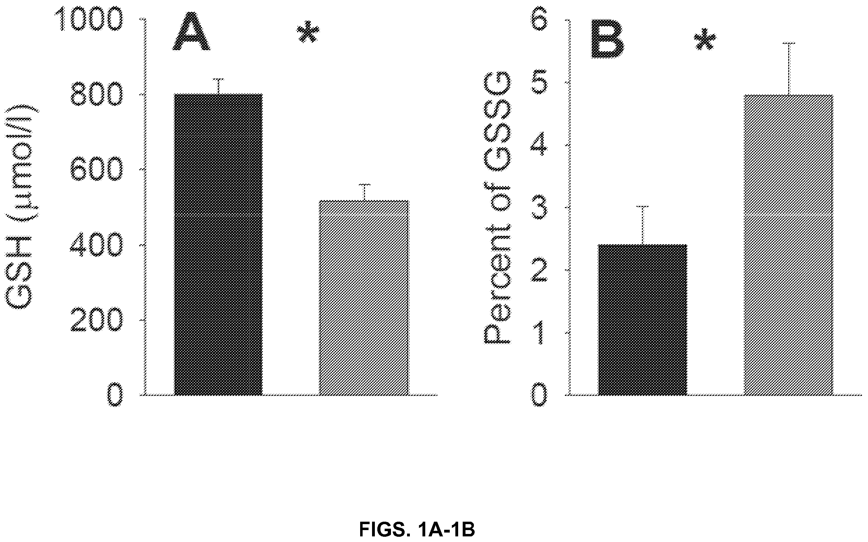

Figures 1A and 1B show (A) decreased blood GSH and (B) elevated GSSG in SZ (grey bars) compared with NC (black bars) (Ballesteros et al. 2013a), according to an embodiment.

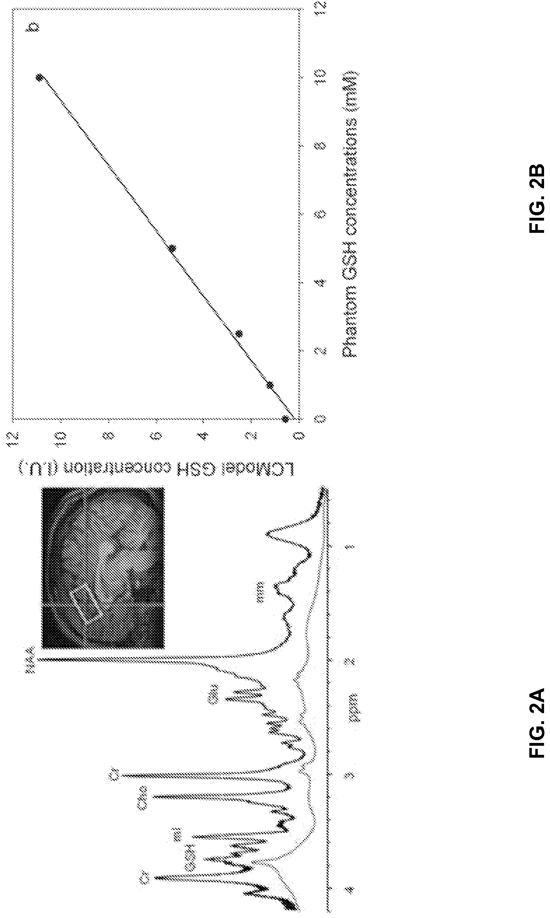

Figures 2A and 2B show (A) MRS GSH spectra (grey) at ACC and (B) the accuracy in measuring GSH, according to an embodiment.

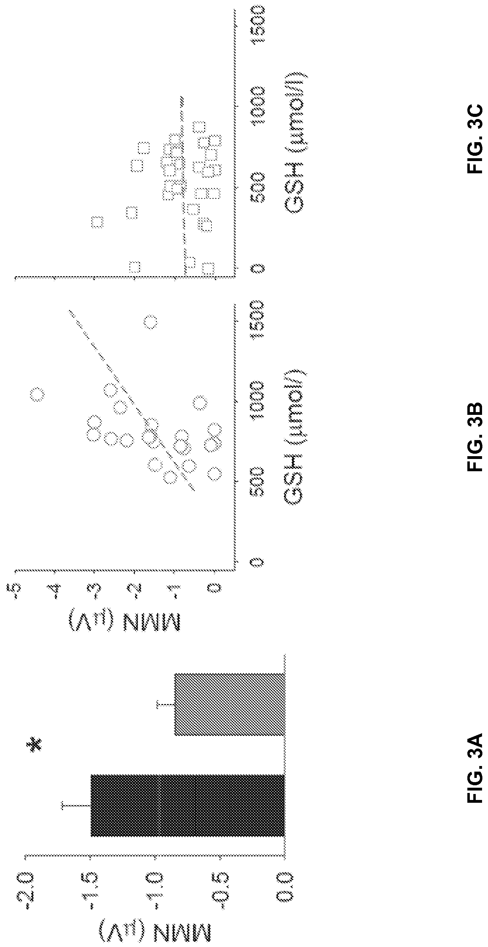

Figures 3A-3C show (A) reduced mismatched negativity (MMN) amplitude in Schizophrenia (SZ) (n = 29) compared with normal control (NC) (n = 25) (*p = 0.015), (B) a significant correlation between MMN and GSH in NC (r = -0.46, p = 0.03), and (C) no such relationship being observed in SZ (r = 0.04, p = 0.85) (Ballesteros et al 2013a), according to an embodiment.

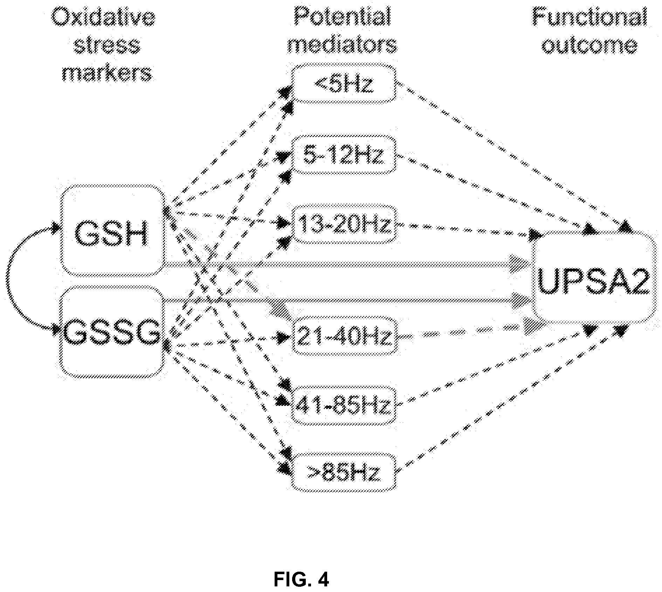

Figure 4 shows a multivariate mediation model where neural oscillatory responses were mediators between GSH and GSSG and community function measure UPSA-2 (California performance-based skills assessment type 2), according to an embodiment. Grey solid lines indicate direct and significant effects of GSH and GSSG on UPSA-2. Grey dotted lines indicate significant indirect effects that the 21-40 Hz oscillatory response was a significant intermediate biomarker linking GSH to functional outcome (UPSA-2).

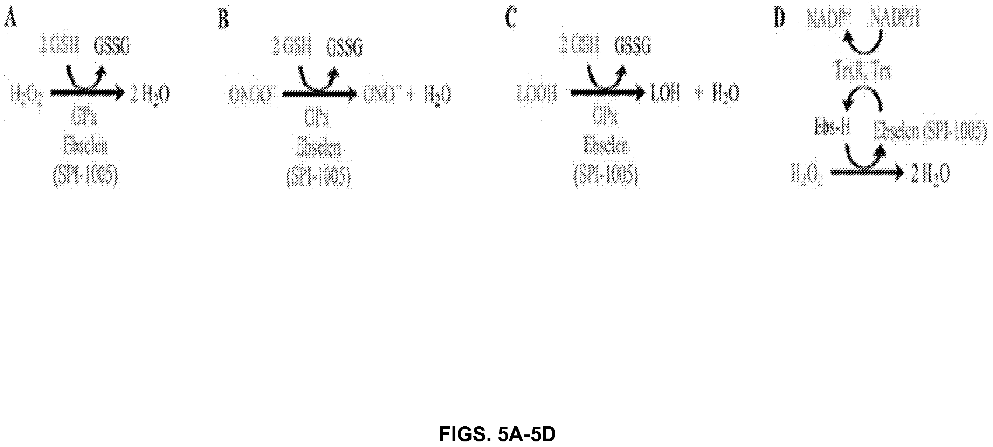

Figures 5A-5D show a proposed mechanisms of antioxidant action of ebselen (adopted from Kil et al., 2007; and Antony et al, 2011), according to an embodiment. (A) Reactive oxygen species (ROS, e.g., H₂O₂) is reduced by GSH and GPx; ebselen assists this process by acting as GPx mimic. (B) Reactive nitrogen species (RNS, e.g., peroxynitrite ONOO⁻) is also reduced by the same GSH system. (C) Lipid hydroperoxides (LOOHs) undergo two-electron reduction to form redox-inert alcohols (LOHs) by GPx, a key membrane/myelin cytoprotection/reparative mechanism with ebselen's effect in this lipid peroxidation redox being well supported. (D) Ebselen is a substrate for the theoredoxin (Trx) system, another key oxidation defense, where ebselen is reduced to ebselen selenol (Ebs-H) by Trx and thoredoxin reductase (TrxR); Ebs-H then serves as an efficient H₂O₂ reductase.

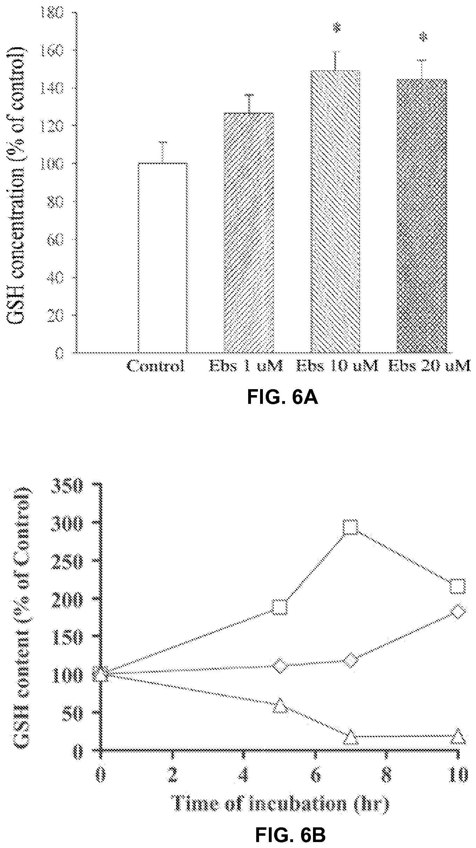

Figures 6A and 6B illustrate ebselen's effect on increasing basal neuronal GSH levels, according to an embodiment. (A) Ebselen treatment increased basal neuronal GSH levels, similar to its on GSH seen under stressed conditions. (B) Neuroprotective effect of ebselen against glutamate-induced GSH depletion and neurotoxity with glutamate decreased cellular GSH level (triangles), ebselen increased basal GSH level (squares), and effect of ebselen and glutamate (diamonds).

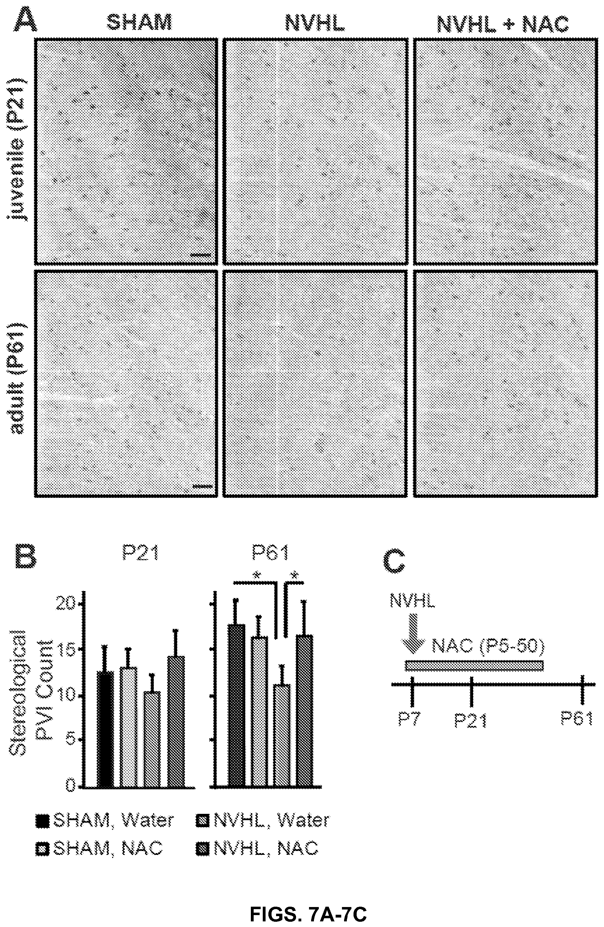

Figures 7A-7C illustrate NVHL blocking the adolescent increase in PV interneuron labeling in the PFC, according to an embodiment. (A) Representative micrographs of prefrontal PV staining in juvenile (P21) and adult (P61) SHAM (placebo), NVHL, and NAC-treated NVHL rats. Scale bar is 80 µm. (B) Bar graphs illustrating PV cell counts using unbiased stereology at P21 (left) and P61 (right) in all four treatment groups. PV cell count increased between P21 and P61 in SHAM, but not in NVHL rats. Juvenile NAC treatment rescued the progression in PV cell numbers in NVHL rats. ANOVA F_{(5,36)} = 4.7, p = 0.002. Age: F_{(1,36)} = 10.2, p = 0.003, Lesion: F_{(1,36)} = 1.36, p = 0.11, Lesion x Age: F_{(1,36)} = 6.7, p = 0.014, Treatment: F_{(1,36)} = 3.9, p = 0.057, Treatment x Age: n.s. (C) Diagram illustrating the time course of NAC treatment and juvenile (P21) and adult (P61) assessments. In this and all other figures, data are expressed as mean ± SEM, *p<0.05.

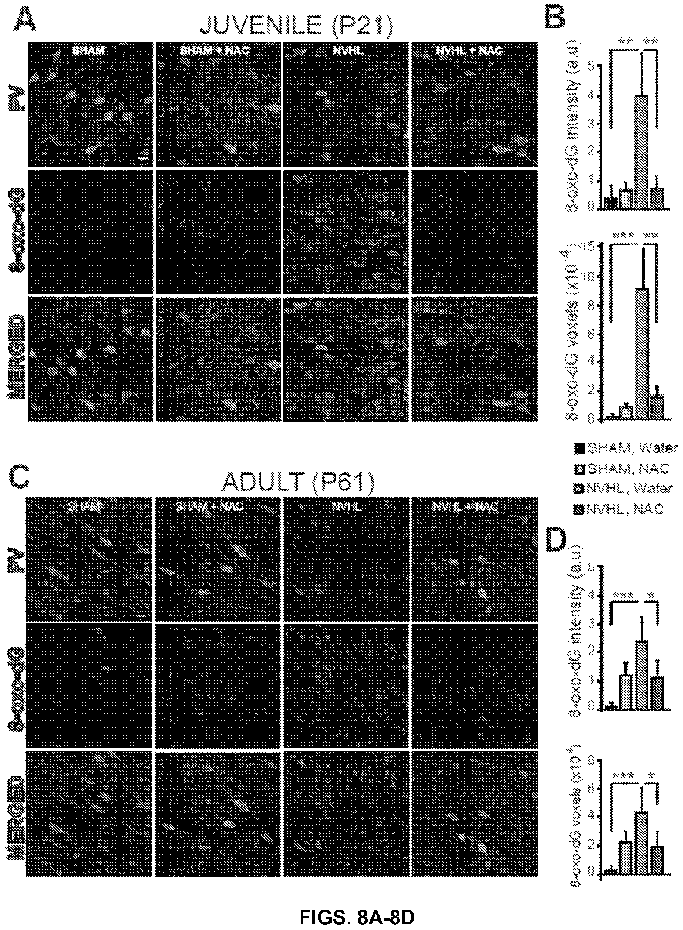

Figures 8A-8D illustrate oxidative stress with 8-oxo-dG in the PFC of NVHL rats, according to an embodiment. (A) Representative micrographs showing double labeling for PV (red) and 8-oxo-dG (green) in the PFC in the four groups at P21. Scale bar is 10 µm. (B) Summary of the data showing that an NVHL causes a massive increase in 8-oxo-dG labeling in the PFC at P21 that is prevented with juvenile NAC treatment. Top graph illustrates 8-oxo-dG fluorescence intensity and the bottom graph quantifies the number of labeled voxels in each group. ANOVA for 8-oxo-dG intensity: F_{(3,14)} = 13.7, p = 0.00002, Lesion F_{(1,14)} = 18.4, p = 0.008, Treatment F_{(1,14)} = 9.6, p = 0.008, Lesion x Treatment F_{(1,14)} = 13.0, p = 0.003. (C) Representative micrographs showing double labeling for PV (red) and 8-oxo-dG (green) in the PFC at P61. Scale bar is 10 µm. (D) Summary of the data showing that the NVHL increases 8-oxo-dG in the PFC at P61, which is prevented with juvenile NAC treatment. ANOVA for 8-oxo-dG intensity: F_{(3,18)}=7.8, p=0.001, Lesion F_{(1,18)} = 12.5, p = 0.002, Treatment F_{(1,18)} = 0.13, p=n.s., Lesion x Treatment F_{(1,18)} = 10.8, p = 0.004.

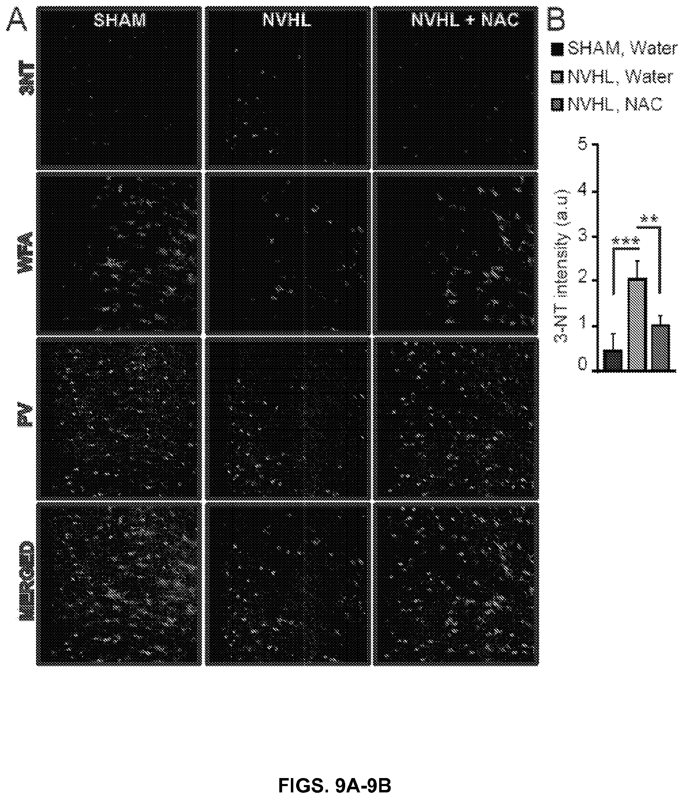

Figures 9A and 9B illustrate oxidative stress in the PFC of adult NVHL rats with 3-NT, according to an embodiment. (A) Representative micrographs showing triple labeling for 3-NT (green), WFA (blue) and PV (red) in the three groups. Scale bar is 100 µm. (B) Summary of the data showing that an NVHL causes a significant increase in 3-NT labeling in the PFC at P61 that is prevented with juvenile NAC treatment. Graph illustrates 3-NT fluorescence intensity in each group. One-way ANOVA for 3-NT intensity revealed a very significant effect of treatment (F_{(2,53)} = 85.2, p < 0.0001). Comparisons between each pairs using Tukey-Kramer also showed significant differences (p < 0.0001) for SHAM versus NVHL and NVHL versus NAC.

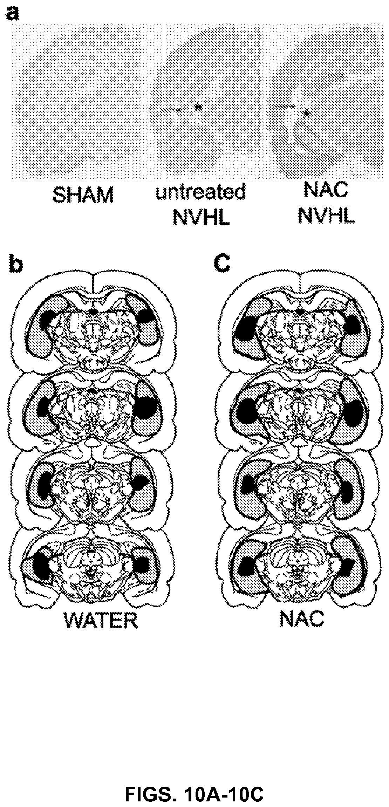

Figures 10A-10C show NVHL lesions, according to an embodiment. (A) Photomicrographs of representative sections from a SHAM (left), an untreated NVHL (middle), and an NAC-treated NVHL (right) brain at the level of the hippocampus. Arrows indicate cell loss in the ventral hippocampus and the stars indicate enlarged ventricles. (B) Cartoons indicating the minimum (black) and maximum extent of lesion in untreated NVHL rats (water) at different rostrocaudal levels throughout the ventral hippocampus. (C) Similar cartoons illustrating the extent of lesion in NAC-treated NVHL rats.

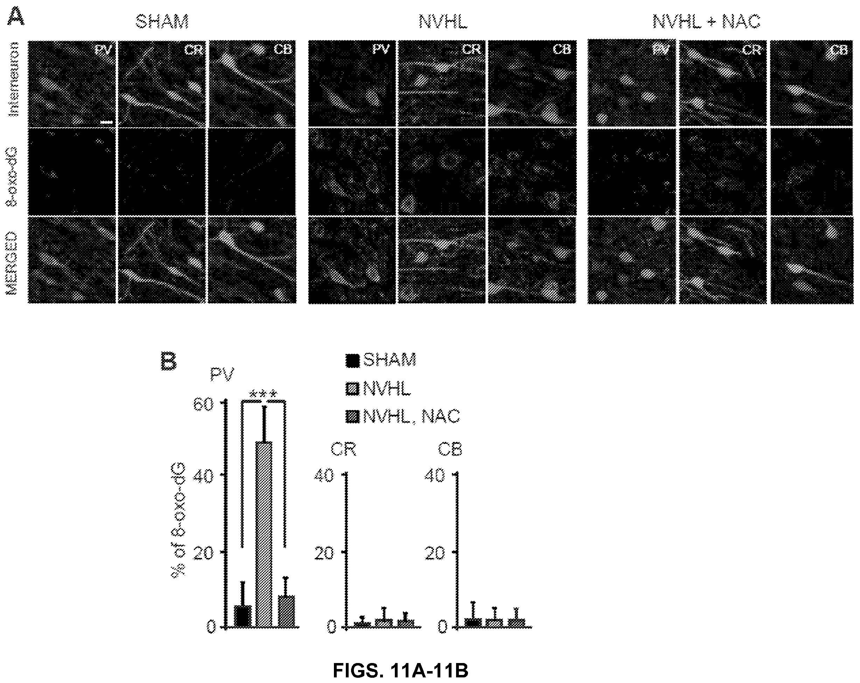

Figures 11A and 11B illustrate that NVHL may cause increased oxidative stress in PV, but not CR and CB interneurons, which is prevented by developmental NAC treatment, according to an embodiment. (A) Micrographs showing 8-oxo-dG labeling (green) of parvalbumin (PV)-, calretinin (CR)- and calbindin (CB)-positive interneurons (red) in the PFC of SHAM, NVHL and NAC- treated NVHL rats. Scale bar is 10 µm. (B) Summary of the data. In PV interneurons, 8- oxo-dG labeling increased following an NVHL lesion, which was prevented with NAC treatment (Treatment: F_{(2,65)} = 212.97, p < 0.0001). ***p < 0.001.

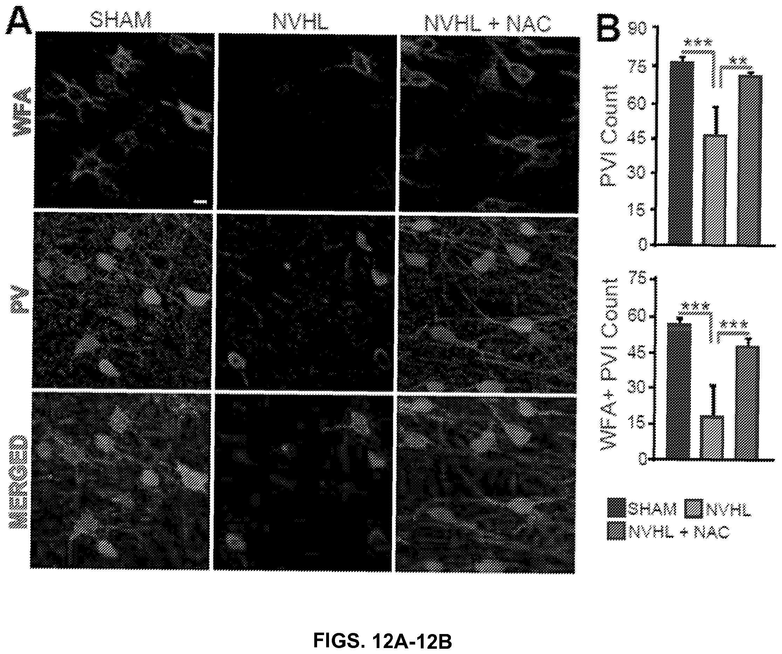

Figures 12A and 12B illustrate that perineuronal nets (PNN) may be reduced in the PFC of adult NVHL rats, but rescued by juvenile NAC treatment, according to an embodiment. (A) Representative micrographs showing double labeling of PV (red) and *Wisteria floribunda agglutinin* (WFA; green), which labels PNN. Scale bar is 10 µm. (B) Plots illustrating PV interneuron (PVI) counts (top) and the number of cells co-labeled with PV and WFA (bottom). PVI count is reduced following an NVHL lesion, and this reduction is prevented with juvenile NAC treatment. (Overall effect: F_{(8,16)}= 3.8, p = 0.01, PVI count: F_{(2,11)} = 15.3, p < 0.0007). The number of WFA PVI decreases in NVHL rats compared to controls, and this reduction is prevented with juvenile NAC treatment (PNN count: F_{(2,11)} = 28.5, p < 0.0001). **p < 0.01, ***p < 0.001.

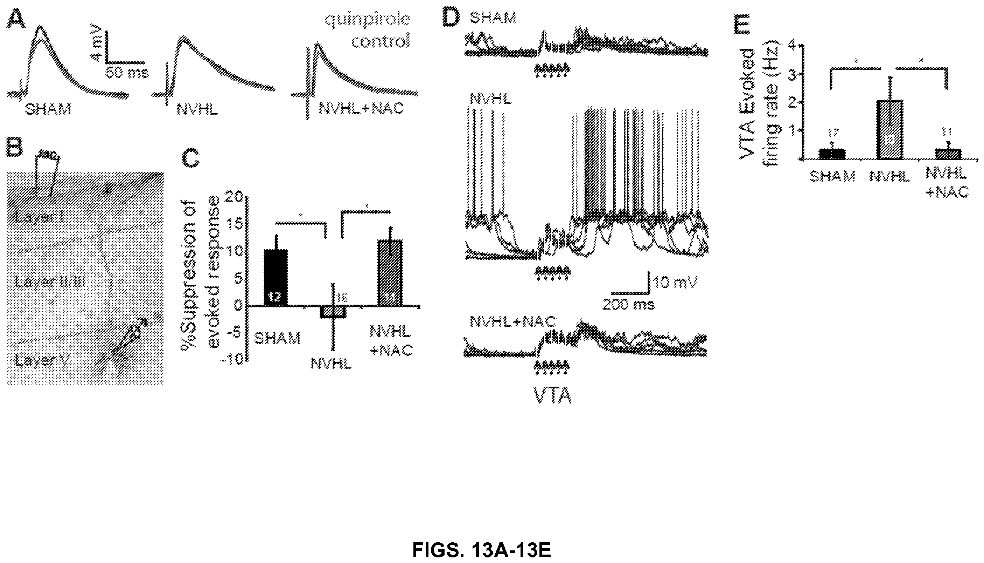

Figures 13A-13E illustrate electrophysiological deficits may be rescued by N-acetyl cysteine (NAC) treatment in NVHL rats, according to an embodiment. (A) Representative traces of excitatory post-synaptic potentials (EPSP) evoked by superficial layer electrical stimulation in adult PFC before (black trace) and after (green trace) bath application of the D2-agonist quinpirole (5 µM). (B) Neurobiotin-filled layer V pyramidal cell in the PFC; the relative position of the bipolar stimulating electrode and the recording electrode are shown schematically. (C) Bar graphs illustrating the magnitude of EPSP attenuation by quinpirole in slices from SHAM, NVHL, and NAC- treated NVHL rats. In sham rats, quinpirole reduces the size of the synaptic response, whereas in NVHL rats this attenuation is absent. NAC treatment during development reverses this deficit in NVHL animals (ANOVA: F_{(2,39)} = 3.328, p = 0.046). (D) Traces from *in vivo* intracellular recordings in PFC pyramidal neurons showing responses to electrical stimulation of the ventral tegmental area (VTA) with trains of 5 pulses at 20 Hz in anesthetized SHAM (top), NVHL (middle), and NAC-treated NVHL (bottom) rats. Each panel is an overlay of 5 traces that illustrate the representative type of response observed in each group, with NVHL showing enhanced firing following VTA stimulation, while firing is sparse in SHAM and NAC-treated NVHL rats. (E) Bar graph illustrating group data for action potential firing in the 500 ms epoch following VTA stimulation in all three groups. ANOVA: F_{(2,37)} = 4.5, p < 0.05; NVHL firing was higher than in shams (post- hoc Tukey's q = 3.9, p < 0.05) and higher than in NAC-treated NVHL rats (post-hoc Tukey's q = 3.6, p < 0.05). In all electrophysiology experiments data from SHAM and NAC-treated SHAM rats were combined as they did not show differences.

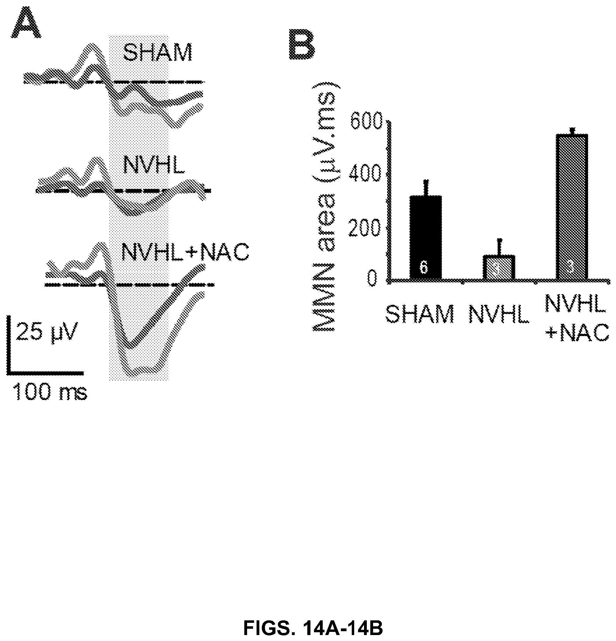

Figures 14A and 14B illustrates that mismatch negativity (MMN) deficits may be rescued by NAC treatment, according to an embodiment. (A) Representative traces of auditory evoked potentials from standard (blue) and deviant (red) stimuli in a sham (n = 6; top), NVHL (n = 3; middle), and NAC-treated NVHL rat (n = 3; bottom). The green box highlights the epoch in which the negativity was measured (35-100 ms following the stimulus). All traces are averages of at least 80 repetitions. (B) Group data comparing MMN measured as the area under the curve in the highlighted region reveal a significant difference among groups (ANOVA: F_{(2,11)} = 9.742; p = 0.006). The data illustrated are averages from 3 different sessions in each rat. A post-hoc comparison between NVHL and NVHL+NAC revealed a significant difference (Bonferroni test; p = 0.005).

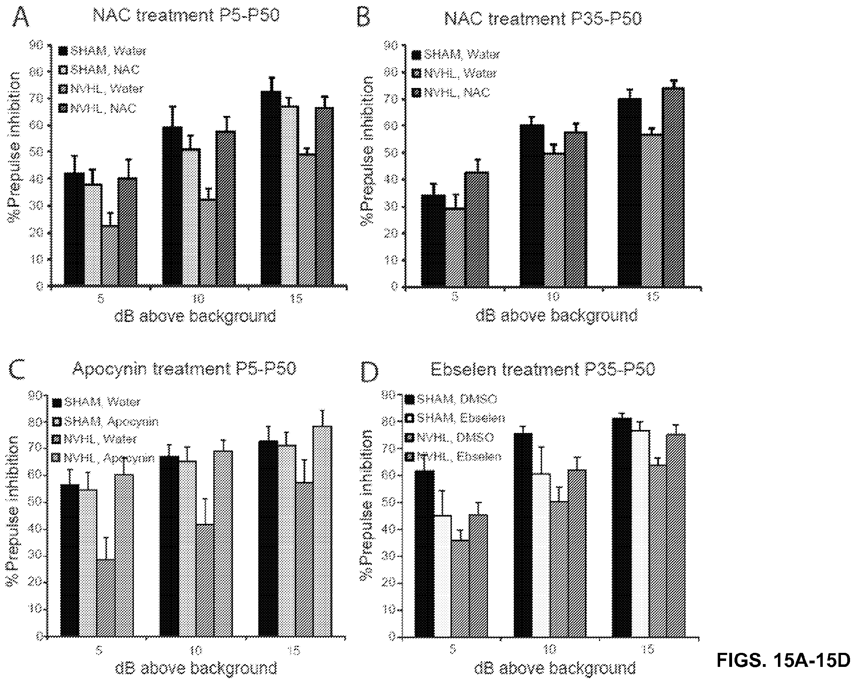

Figures 15A-15D illustrate that prepulse inhibition deficits may be rescued with antioxidant treatment, according to an embodiment. (A) Prepulse inhibition deficits were observed in NVHL rats when challenged with apomorphine (0.1 mg/kg, i.p.). This deficit was completely reversed with juvenile NAC treatment (Lesion: F_{(1,42)} = 3.529 p = 0.067, Treatment: F_{(1,42)} =1 .644, p = 0.207, Lesion x Treatment: F_{(1,42)} = 5.730, p = 0.021). n = 12-16, * p < 0.05 compared to NVHL. (B) In another group of rats, NAC was administered starting at P35, stopped at P50, and the rats tested for PPI at P61. The bar graph illustrates PPI at three different prepulse intensities in this group with adolescent NAC treatment. ANOVA: group effect F_{(2,28)} = 3.364, p < 0.045; post-hoc tests revealed only a trend for a difference in PPI in NVHL compared to SHAM (LSD, p = 0.069), and a significant difference between NVHL and NAC-treated NVHL (LSD, p = 0.016). (C) Some animals received Ebselen from P35 and were tested for PPI at P61. There was a significant lesion effect (F_{(1,28)} = 7.11; p = 0.013) and a significant lesion status by treatment interaction (F_{(1,28)} = 7.09; p = 0.013). (D) Another set of animals received apocynin and were tested for PPI. We observed a significant lesion by treatment interaction (F_{(1,25)} = 4.8; p = 0.038).

### DETAILED DESCRIPTION

Embodiments in the present disclosure relate to novel combinations of at least two compounds, the first compound comprising a glutathione peroxidase modulator and the second compound is an antipsychotic agent, for the treatment, amelioration, prevention or inhibition of numerous conditions, including but not limited to GPx mediated disorders, heightened oxidative stress, schizophrenia, bi-polar disorder, depression, mania, anxiety disorders or related psychotic disorders, tardive dyskinesia, and associated symptoms or complications thereof.

Representative compounds of the novel combination are described throught the specification and claims.

In some embodiments, the antipsychotic agent is selected from the group consisting of glutathione, glutathione prodrugs, cysteine prodrugs, Chlorpromazine (Thorazine), Haloperidol (Haldol), Perphenazine, Fluphenazine, Risperidone (Risperdal), Olanzapine (Zyprexa), Quetiapine (Seroquel), Ziprasidone (Geodon), Aripiprazole (Abilify), Paliperidone (Invega), Lurasidone (Latuda) and combinations thereof.

In some embodiments, a glutathione peroxidase modulator comprises a compound selected from the group consisting of gluthione peroxidase mimic compounds, glutathione, glutathione prodrugs, and cysteine prodrugs.

In some embodiments, a representative compound of a gluthione peroxidase mimic compound comprises ebselen, (2-phenyl-1,2-benzisoselenazol-3(2H)-one) with an empirical formula C₁₃H₉NOSe, molecular weight 274.2 and a formula of:

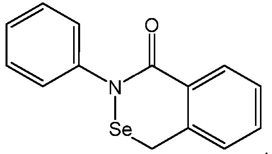

In some embodiments, gluthione peroxidase mimic compounds comprise 2,2'-diseleno-bis-β-cyclodextrin and 6A,6B-diseleninic acid-6A',6B'-selenium bridged β-cyclodextrin.

Glutathione peroxidase mimics, like glutathione peroxidase, reduce reactive oxygen species by the binding of free radicals to its Se moiety. By reacting with glutathione, glutathione peroxidase mimics limit free radical toxicity, thus exhibiting strong activity against peroxynitrite. Ebselen, a glutathione peroxidase mimic, reduces cytochrome C release from mitochondria and nuclear damage during lipid peroxidation, thus attenuating neuronal apoptosis associated with oxidative stress. Agents that reduce the activity of reactive oxygen species can ameliorate the deleterious effects of heightened oxidative stress and diseases caused by such stress, including but not limited to heightened oxidative stress, schizophrenia, bi-polar disorder, depression, mania, anxiety disorders or related psychotic disorders, tardive dyskinesia, and associated symptoms or complications thereof.

Oxidative stress in schizophrenia patients is associated with a glutathione (GSH) deficit. FIGS. 1A and 1B illustrate fasting blood GSH and glutathione disulfide (GSSG) in SZs (n = 29) and normal control patients (n = 25). GSH was lower (p < 0.001) and GSSG was higher (p = 0.023) in SZ, whereas exogenous sources in diet, smoking, or medications, and were not found to cause the abnormal GSH redox state in SZ, suggesting an intrinsic redox abnormality. Reduced GSH was also found in patients on clozapine (p = 0.005) or other antipsychotics (p < 0.001) (Ballesteros et al., 2013a).

In some embodiments, representative glutathione produgs comprise compounds of the formula: 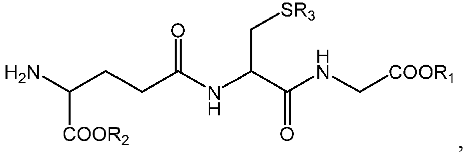 wherein
R₁ is H, methyl, ethyl, or isopropyl;
R₂ is H, or ethyl; and
R₃ is H, acetyl, phenylacetyl,

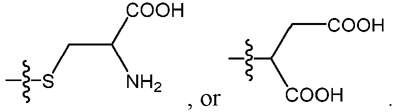

In some embodiments, a representative cysteine prodrug comprises N-acetyl cysteine (NAC) with a formula of:

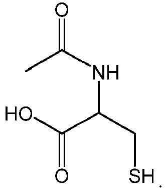

Some embodiments of cysteine prodrugs comprise N,N'-diacetyl-cysteine, N-acetyl cysteine amide, NAC esters (alkyl esters, glycolamide esters and acycloxymethyl esters), S-allyl cysteine, S-methyl cysteine, S-ethyl cysteine, S-propyl cysteine, or compounds of the formula: 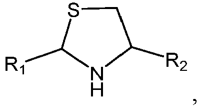 wherein
R₁ is H, oxo, methyl, ethyl, n-propyl, n-pentyl, phenyl, -(CHOH)ₙCH₂OH and wherein n is 1-5, or 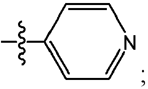 and
R₂ is H or -COOH.

Some embodiments of cysteine prodrugs comprise 2-substituted thiazolidine-4-carboxylic acids with aldose monosaccarides, such as glyceraldehyde, arabinose, lyxose, ribose, xylose, galactose, glucose, and mannose.

Another aspect of the present disclosure features a pharmaceutical composition comprising a novel combination and at least one pharmaceutically acceptable carrier.

The present disclosure further features a process for making a pharmaceutical composition comprising admixing any of the compounds of the novel combination and a pharmaceutically acceptable carrier.

Yet another aspect of the present disclosure features a method of treating a subject suffering from or diagnosed with a disease, disorder, or condition mediated by GPx activity, comprising administering to the subject a therapeutically effective amount of a novel combination. Such disease, disorder, or condition can include, but is not limited to heightened oxidative stress, schizophrenia, bi-polar disorder, depression, mania, anxiety disorders or related psychotic disorders, tardive dyskinesia, and associated symptoms or complications thereof.

Another aspect of the present disclosure features a method for reducing the side effects of administering an antipsychotic agent by co-administering a gluthione peroxidase mimic compound with the antipsychotic agent. In particular, reducing the side effects of administering an antipsychotic agent by co-administering ebselen with the antipsychotic agent. Even more particular, reducing the side effects of administering Chlorpromazine (Thorazine), Haloperidol (Haldol), Perphenazine, Fluphenazine, Risperidone (Risperdal), Olanzapine (Zyprexa), Quetiapine (Seroquel), Ziprasidone (Geodon), Aripiprazole (Abilify), Paliperidone (Invega), Lurasidone (Latuda) or combinations thereof by co-administering ebselen. In some embodiments, the side effects of administering an antipsychotic agent comprise tardive dyskinesia and other complications of administering an antipsychotic agent to a patient at high doses or over a long term.

In a further embodiment, a method for treating or ameliorating a GPx mediated condition in a subject in need thereof comprises administering to the subject a therapeutically effective amount of the novel combination, wherein the therapeutically effective amount of each compound in the combination is from about 0.1 mg/dose to about 5 g/dose. In particular, the therapeutically effective amount of each compound in the combination is from about 0.5 mg/dose to about 1000 mg/dose. More particularly, the therapeutically effective amount of each compound in the combination is from about 1 mg/dose to about 100 mg/dose. In a further embodiment, the number of doses per day of the combination is from 1 to 3 doses. In a further embodiment, the therapeutically effective amount of each compound in the combination is from about 0.001 mg/kg/day to about 30 mg/kg/day. More particularly, the therapeutically effective amount of each compound in the combination is from about 0.01 mg/kg/day to about 2 mg/kg/day.

In a further embodiment, a method for preventing or inhibiting the progression of an GPx mediated condition in a subject in need thereof comprises administering to the subject a therapeutically effective amount of the combination, wherein the therapeutically effective amount of each compound in the combination is from about 0.1 mg/dose to about 5 g/dose. In particular, the therapeutically effective amount of each compound in the combination is from about 1 mg/dose to about 100 mg/dose. In a further embodiment, the number of doses per day of the combination is from 1 to 3 doses. In a further embodiment, the therapeutically effective amount of each compound in the combination is from about 0.001 mg/kg/day to about 30 mg/kg/day. More particularly, the therapeutically effective amount of each compound in the combination is from about 0.01 mg/kg/day to about 2 mg/kg/day.

### Definitions/Terms

In general, terms used in the claims and the specification are intended to be construed as having the plain meaning understood by a person of ordinary skill in the art. Certain terms are defined below to provide additional clarity. In case of conflict between the plain meaning and the provided definitions, the provided definitions are to be used. Terms used in the claims and specification are defined as set forth below unless otherwise specified or by their usage throughout this disclosure.

Unless otherwise noted, "alkyl" as used herein, whether used alone or as part of a substituent group, refers to a saturated, branched, or straight-chain monovalent hydrocarbon radical derived by the removal of one hydrogen atom from a single carbon atom of a parent alkane. Typical alkyl groups include, but are not limited to, methyl; ethyls such as ethanyl; propyls such as propan-1-yl, propan-2-yl, cyclopropan-1-yl; butyls such as butan-1-yl, butan-2-yl, 2-methyl-propan-1-yl, 2-methyl-propan-2-yl, cyclobutan-1-yl and the like. In preferred embodiments, the alkyl groups are C1-6alkyl, with C1-3 being particularly preferred. "Alkoxy" radicals are oxygen ethers formed from the previously described straight or branched chain alkyl groups. In some embodiments, the alkyl or alkoxy are independently substituted with one to five, preferably one to three groups including, but not limited to, oxo, amino, alkoxy, carboxy, heterocyclyl, hydroxyl, and halo (F, Cl, Br, or I).

The term "aryl," as used herein, refers to aromatic groups comprising a stable six-membered monocyclic, or ten-membered bicyclic or fourteen-membered tricyclic aromatic ring system which consists of carbon atoms. Examples of aryl groups include, but are not limited to, phenyl or naphthalenyl. In some embodiments, "aryl" is substituted. For instance, "aryl" can be substituted with, e.g., optionally substituted C1-6alkyl, C2-6alkenyl, C2-6alkynyl, halo, hydroxyl, -CN, -C(O)OH, -C(O)O-C1-4alkyl, -C(O)NR'R", -SR', -OR', -C(O)R', -N(R')(R"), -S(O)2-R', and -S(O)2-N(R')(R"), wherein R' and R" are independently selected from H, C1-6-alkyl, aryl, heteroaryl, and/or heterocyclyl.

The term "heterocyclyl" or "heterocycle" is a 3- to 8-member saturated, or partially saturated single or fused ring system which consists of carbon atoms and from 1 to 6 heteroatoms selected from N, O and S. The heterocyclyl group may be attached at any heteroatom or carbon atom which results in the creation of a stable structure. Example of heterocyclyl groups include, but are not limited to, 2-imidazoline, imidazolidine; morpholine, oxazoline, 2-pyrroline, 3-pyrroline, pyrrolidine, pyridone, pyrimidone, piperazine, piperidine, indoline, tetrahydrofuran, 2-pyrroline, 3-pyrroline, 2-imidazoline, 2-pyrazoline, indolinone. In some embodiments, "heterocyclyl" or "heterocycle" are independently substituted. For instance, "heterocyclyl" or "heterocycle" can be substituted with, e.g., optionally substituted C1-6alkyl, C2-6alkenyl, C2-6alkynyl, halo, hydroxyl, -CN, -C(O)OH, -C(O)O-C1-4alkyl, -C(O)NR'R"-OR', -SR'-C(O)R', -N(R')(R"), -S(O)2-R', and -S(O)2-N(R')(R"), wherein R' and R" are independently selected from H, C1-6-alkyl, aryl, heteroaryl, and/or heterocyclyl.

The term "oxo" whether used alone or as part of a substituent group refers to an O= to either a carbon or a sulfur atom. For example, phthalimide and saccharin are examples of compounds with oxo substituents.

The term "cis-trans isomer" refers to stereoisomeric olefins or cycloalkanes (or hetero-analogues) which differ in the positions of atoms (or groups) relative to a reference plane: in the cis-isomer the atoms are on the same side; in the trans-isomer they are on opposite sides.

The term "substituted" refers to a radical in which one or more hydrogen atoms are each independently replaced with the same or different substituent(s).

With reference to substituents, the term "independently" means that when more than one of such substituent is possible, such substituents may be the same or different from each other.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques known in the art as well as those methods set forth herein.

Methods are known in the art for determining effective doses for therapeutic and prophylactic purposes for the disclosed pharmaceutical compositions or the disclosed drug combinations, whether or not formulated in the same composition. For therapeutic purposes, the term "therapeutically effective amount" as used herein, means that amount of each active compound or pharmaceutical agent, alone or in combination, that elicits the biological or medicinal response in a tissue system, animal or human that is being sought by a researcher, veterinarian, medical doctor or other clinician, which includes alleviation of the symptoms of the disease or disorder being treated. For prophylactic purposes (i.e., inhibiting the onset or progression of a disorder), the term "therapeutically effective amount" refers to that amount of each active compound or pharmaceutical agent, alone or in combination, that treats or inhibits in a subject the onset or progression of a disorder as being sought by a researcher, veterinarian, medical doctor or other clinician. Thus, the present invention provides combinations of two or more drugs wherein, for example, (a) each drug is administered in an independently therapeutically or prophylactically effective amount; (b) at least one drug in the combination is administered in an amount that is sub-therapeutic or sub-prophylactic if administered alone, but is therapeutic or prophylactic when administered in combination with the second or additional drugs according to the invention; or (c) both (or more) drugs are administered in an amount that is sub-therapeutic or sub-prophylactic if administered alone, but are therapeutic or prophylactic when administered together.

The term "pharmaceutically acceptable salt" refers to non-toxic pharmaceutically acceptable salts (Ref. International J. Pharm., 1986, 33, 201-217; J. Pharm. Sci., 1997 (January), 66, 1, 1). Other salts well known to those in the art may, however, be useful in the preparation of compounds according to this invention or of their pharmaceutically acceptable salts. Representative organic or inorganic acids include, but are not limited to, hydrochloric, hydrobromic, hydriodic, perchloric, sulfuric, nitric, phosphoric, acetic, propionic, glycolic, lactic, succinic, maleic, fumaric, malic, tartaric, citric, benzoic, mandelic, methanesulfonic, hydroxyethanesulfonic, benzenesulfonic, oxalic, pamoic, 2-naphthalenesulfonic, p-toluenesulfonic, cyclohexanesulfamic, salicylic, saccharinic or trifluoroacetic acid. Representative organic or inorganic bases include, but are not limited to, basic or cationic salts such as benzathine, chloroprocaine, choline, diethanolamine, ethylenediamine, meglumine, procaine, aluminum, calcium, lithium, magnesium, potassium, sodium and zinc.

The term "composition" is intended to encompass a product comprising the specified ingredients in the specified amounts, as well as any product which results, directly or indirectly, from combinations of the specified ingredients in the specified amounts.

The term "subject" encompasses an organism, an animal, including a mammal, human or non-human, male or female, who is the object of treatment, observation, clinical trial or experiment. The subject can be a human patient. The term "human" generally refers to *Homo sapiens.* The term "mammal" as used herein includes but is not limited to a human, non-human primate, mouse, rat, guinea pig, chinchilla and monkey. Mammals other than humans can be advantageously used as subjects that represent animal models of, e.g., hearing loss, schizophrenia, bipolar disorders, and/or any other psychotic disorder.

The term "percent identity" or "percent sequence identity," in the context of two or more nucleic acid or polypeptide sequences, refer to two or more sequences or subsequences that have a specified percentage of nucleotides or amino acid residues that are the same, when compared and aligned for maximum correspondence, as measured using one of the sequence comparison algorithms described below (e.g., BLASTP and BLASTN or other algorithms available to persons of skill) or by visual inspection. Depending on the application, the percent "identity" can exist over a region of the sequence being compared, e.g., over a functional domain, or, alternatively, exist over the full length of the two sequences to be compared.

For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally Ausubel et al., infra).

One example of an algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information website.

Psychotic disorders, diseases, or prodromal conditions include, but are not limited to heightened oxidative stress, schizophrenia, bi-polar disorder, depression, mania, anxiety disorders or related psychotic disorders, tardive dyskinesia, and associated symptoms or complications thereof.

The term "statistically significant" is defined as the probability that a result is not caused by random chance.

It must be noted that, as used in the specification and the appended claims, the singular forms "a," "an," and "the" include plural referents unless the context clearly dictates otherwise.

Abbreviations or acronyms used in the throughout the specification include:
- ACC: Anterior cingulate cortex
- AEC: Adverse event checklist
- BPRS: Brief psychistric rating scale
- CAT: Catalase
- CDSS: Calgary depression scale for schizophrenia
- C-SSRS: Columbia suicide severity rating scale
- EOS: End of study
- GSH: The reduced form of glutathione
- GSSG: The oxidized form of glutathione
- GPx: glutathione peroxidases
- GR: Glutathione reductase
- MCCB: MATRICS consensus cognitive battery
- MMN: Mismatch negativity
- MRS: Magnetic resonance spectroscopy
- NAC: N-acetyl-cysteine
- NC: Normal controls
- NMDAR: N-methyl-D-aspartate receptors
- POC: Proof of concept
- Redox: Reduction/oxidation
- RNS: Reactive nitrogen species
- ROS: Reactive oxygen species
- SOD: Superoxide dismutase
- SPI: Sound Pharmaceutical Inc
- SZ: Schizophrenia patients or Schizophrenia
- UPSA: California performance-based skills assessment
- h or hr (hour(s))
- LCMS (high pressure liquid chroatography with mass spectrometer)
- Me (methyl)
- Mg (milligram)
- rt or RT (room temperature)
- TLC (thin layer chromatography)

### References

Antony S, Bayse CA. Modeling the mechanism of the glutathione peroxidase mimic ebselen. Inorg Chem. 2011;50:12075-12084.

Albers, L. J., Ozdemir, V., Marder, S. R., Raggi, M. A., Aravagiri, M., Endrenyi, L., & Reist, C. (2005). Low-dose fluvoxamine as an adjunct to reduce olanzapine therapeutic dose requirements: a prospective dose-adjusted drug interaction strategy. Journal of clinical psychopharmacology, 25(2), 170-174.

Ballesteros A, Jiang P, Summerfelt A, Du X, Chiappelli J, O'Donnell P, Kochunov P, Hong LE. (2013a) No evidence of exogenous origin for the abnormal glutathione redox state in schizophrenia. Schizophre Res. 2013;146:184-189

Ballesteros A, Summerfelt A, Du X, Jiang P, Chiappelli J, Tagamets, M, O'Donnell P, Kochunov P, Hong LE. (2013b) Electrophysiological Intermediate Biomarkers for Oxidative Stress in Schizophrenia. Clin Neurophysiol. 2013 Jun 30. doi:pii: S1388-2457(13)00695-0. 10.1016/j.clinph.2013.05.021. [Epub ahead of print]

Behrens, M.M., Ali, S.S., Dao, D.N., Lucero, J., Shekhtman, G., Quick, K.L., and Dugan, L.L. (2007). Ketamine- induced loss of phenotype of fast-spiking interneurons is mediated by NADPH-oxidase. Science 318, 1645-1647.

Berk, M., Copolov, D., Dean, O., Lu, K., Jeavons, S., Schapkaitz, I., Anderson-Hunt, M., Judd, F., Katz, F., Katz, P., et al. (2008). N-acetyl cysteine as a glutathione precursor for schizophrenia--a double-blind, randomized, placebo-controlled trial. Biological psychiatry 64, 361-368.

Bitanihirwe, B.K., and Woo, T.U. (2011). Oxidative stress in schizophrenia: an integrated approach. Neuroscience and biobehavioral reviews 35, 878-893.

Cabungcal, J. H., Counotte, D. S., Lewis, E., Tejeda, H. A., Piantadosi, P., Pollock, C., Calhoon, G. G., Sullivan, E., Presgraves, E., Kil, J., Hong, L. E., Cuenod, M., Do, K. Q., & O'Donnell, P. (2014). Juvenile antioxidant treatment prevents adult deficits in a developmental model of schizophrenia. Neuron, 83(5), 1073-1084.

Cabungcal, J.H., Nicolas, D., Kraftsik, R., Cuenod, M., Do, K.Q., and Hornung, J.P. (2006). Glutathione deficit during development induces anomalies in the rat anterior cingulate GABAergic neurons: Relevance to schizophrenia. Neurobiology of disease 22, 624-637.

Cabungcal, J.H., Steullet, P., Kraftsik, R., Cuenod, M., and Do, K.Q. (2013a). Early-life insults impair parvalbumin interneurons via oxidative stress: reversal by N-acetylcysteine. Biological psychiatry 73, 574-582.

Cabungcal, J.H., Steullet, P., Morishita, H., Kraftsik, R., Cuenod, M., Hensch, T.K., and Do, K.Q. (2013b). Perineuronal nets protect fast-spiking interneurons against oxidative stress. Proceedings of the National Academy of Sciences of the United States of America 110, 9130-9135.

Carlen M, Meletis K, Siegle JH, Cardin JA, Futai K, Vierling-Claassen D, Ruhlmann C, Jones SR, Deisseroth K, Sheng M, Moore CI, Tsai LH. A critical role for NMDA receptors in parvalbumin interneurons for gamma rhythm induction and behavior. Mol Psychiatry. 2012; 17:537-548.

Carmeli, C., Knyazeva, M.G., Cuenod, M., and Do, K.Q. (2012). Glutathione precursor N-acetyl-cysteine modulates EEG synchronization in schizophrenia patients: a double-blind, randomized, placebo-controlled trial. PloS one 7, e29341.

Chambers, R.A., and Lipska, B.K. (2011). A method to the madness: producing the neonatal ventral hippocampal lesion rat model of schizophrenia. In Animal Models of Schizophrenia and Related Disorders, P. O'Donnell, ed. (New York, Humana Press), pp. 1-24.

Cho RY, Konecky RO, Carter CS. Impairments in frontal cortical gamma synchrony and cognitive control in schizophrenia. Proc Natl Acad Sci U S A. 2006; 103:19878-19883.

Choi YB, Lipton SA. Redox modulation of the NMDA receptor. Cell Mol Life Sci. 2000; 57:1535-1541.

Daiber A, Zou MH, Bachschmid M, Ullrich V. Ebselen as a peroxynitrite scavenger in vitro and ex vivo. Biochem Pharmacol. 2000; 59:153-160.

das Neves Duarte, J.M., Kulak, A., Gholam-Razaee, M.M., Cuenod, M., Gruetter, R., and Do, K.Q. (2012). N-acetylcysteine normalizes neurochemical changes in the glutathione-deficient schizophrenia mouse model during development. Biological psychiatry 71, 1006-1014.

Dietrich-Muszalska A, Olas B. Isoprostenes as indicators of oxidative stress in schizophrenia. World J Biol Psychiatry. 2009; 10:27-33.

Do, K.Q., Cabungcal, J.H., Frank, A., Steullet, P., and Cuenod, M. (2009). Redox dysregulation, neurodevelopment, and schizophrenia. Curr Opin Neurobiol 19, 220-230.

Do, K.Q., Trabesinger, A.H., Kirsten-Kruger, M., Lauer, C.J., Dydak, U., Hell, D., Holsboer, F., Boesiger, P., and Cuenod, M. (2000). Schizophrenia: glutathione deficit in cerebrospinal fluid and prefrontal cortex in vivo. The European journal of neuroscience 12, 3721-3728.

Ehrlichman, R.S., Gandal, M.J., Maxwell, C.R., Lazarewicz, M.T., Finkel, L.H., Contreras, D., Turetsky, B.I., and Siegel, S.J. (2009). N-methyl-d-aspartic acid receptor antagonist-induced frequency oscillations in mice recreate pattern of electrophysiological deficits in schizophrenia. Neuroscience 158, 705-712.

Feleder, C., Tseng, K.Y., Calhoon, G.G., and O'Donnell, P. (2010). Neonatal intrahippocampal immune challenge alters dopamine modulation of prefrontal cortical interneurons in adult rats. Biological psychiatry 67, 386-392.

Fischer H, Terlinden R, Lohr JP, Romer A. A novel biologically active selenoorganic compound. VIII. Biotransformation of ebselen. Xenobiotica. 1988; 18:1347-1359.

Gawryluk, J.W., Wang, J.F., Andreazza, A.C., Shao, L., and Young, L.T. (2011). Decreased levels of glutathione, the major brain antioxidant, in post-mortem prefrontal cortex from patients with psychiatric disorders. The international journal of neuropsychopharmacology / official scientific j ournal of the Collegium Internationale Neuropsychopharmacologicum 14, 123-130.

Geyer, M.A., and Braff, D.L. (1987). Startle habituation and sensorimotor gating in schizophrenia and related animal models. Schizophrenia bulletin 13, 643-668.

Giovanoli, S., Engler, H., Engler, A., Richetto, J., Voget, M., Willi, R., Winter, C., Riva, M.A., Mortensen, P.B., Schedlowski, M., and Meyer, U. (2013). Stress in puberty unmasks latent neuropathological consequences of prenatal immune activation in mice. Science 339, 1095-1099.

Gonzalez-Burgos G, Lewis DA. NMDA receptor hypofunction, parvalbumin-positive neurons, and cortical gamma oscillations in schizophrenia. Schizophr Bull. 2012; 38:950-957.

Gysin, R., Kraftsik, R., Sandell, J., Bovet, P., Chappuis, C., Conus, P., Deppen, P., Preisig, M., Ruiz, V., Steullet, P., et al. (2007). Impaired glutathione synthesis in schizophrenia: convergent genetic and functional evidence. Proceedings of the National Academy of Sciences of the United States of America 104, 16621-16626.

Haddad, J.J. (2002). Antioxidant and prooxidant mechanisms in the regulation of redox(y)-sensitive transcription factors. Cellular signalling 14, 879-897.

Hartig, W., Brauer, K., Bigl, V., and Bruckner, G. (1994). Chondroitin sulfate proteoglycan-immunoreactivity of lectin-labeled perineuronal nets around parvalbumin-containing neurons. Brain research 635, 307-311.

Hensch, T.K. (2005). Critical period plasticity in local cortical circuits. Nature reviews Neuroscience 6, 877-888. Javitt, D.C., Doneshka, P., Zylberman, I., Ritter, W., and Vaughan, H.G., Jr. (1993). Impairment of early cortical processing in schizophrenia: an event-related potential confirmation study. Biological psychiatry 33, 513-519.

Herin GA, Du S, Aizenman E. The neuroprotective agent ebselen modifies NMDA receptor function via the redox modulatory site. J Neurochem. 2001; 78:1307-1314.

Hong LE, Summerfelt A, McMahon R, Adami H, Francis G, Elliott A, Buchanan RW, Thaker GK. Evoked gamma band synchronization and the liability for schizophrenia. Schizophr Res. 2004; 70:293-302.

Imai H, Masayasu H, Dewar D, Graham DI, Macrae IM. Ebselen protects both gray and white matter in a rodent model of focal cerebral ischemia. Stroke. 2001; 32:2149-2154.

Javitt DC, Doneshka P, Zylberman I, Ritter W, Vaughan HG, Jr. Impairment of early cortical processing in schizophrenia: an event-related potential confirmation study. Biol Psychiatry. 1993; 33:513-519.

Javitt DC, Grochowski S, Shelley AM, Ritter W. Impaired mismatch negativity (MMN) generation in schizophrenia as a function of stimulus deviance, probability, and interstimulus/interdeviant interval. Electroencephalogr Clin Neurophysiol. 1998; 108:143-153.

Javitt DC, Doneshka P, Grochowski S, Ritter W, Jonides J, Smith EE, Koeppe RA, Awh E, Minoshima S, Mintun MA, Just MA, Carpenter PA, Huerta MF, Krubitzer LA, Kaas JH, Keating EG, Pierre A, Chopra S. Impaired mismatch negativity generation reflects widespread dysfunction of working memory in schizophrenia. Arch Gen Psychiatry. 1996; 76:637-641.

Johnson, A.W., Jaaro-Peled, H., Shahani, N., Sedlak, T.W., Zoubovsky, S., Burruss, D., Emiliani, F., Sawa, A., and Gallagher, M. (2013). Cognitive and motivational deficits together with prefrontal oxidative stress in a mouse model for neuropsychiatric illness. Proceedings of the National Academy of Sciences of the United States of America 110, 12462-12467.

Jones, D.P., and Go, Y.M. (2010). Redox compartmentalization and cellular stress. Diabetes Obes Metab 12 Suppl 2, 116-125.

Kano S, Colantuoni C, Han F, Zhou Z, Yuan Q, Wilson A, Takayanagi Y, Lee Y, Rapoport J, Eaton W, Cascella N, Ji H, Goldman D, Sawa A. Genome-wide profiling of multiple histone methylations in olfactory cells: further implications for cellular susceptibility to oxidative stress in schizophrenia. Mol Psychiatry. 2013;18:740-742.

Kasai, H. (1997). Analysis of a form of oxidative DNA damage, 8-hydroxy-2'-deoxyguanosine, as a marker of cellular oxidative stress during carcinogenesis. Mutat Res 387, 147-163.

Kil, J., Pierce, C., Tran, H., Gu, R., and Lynch, E.D. (2007). Ebselen treatment reduces noise induced hearing loss via the mimicry and induction of glutathione peroxidase. Hearing research 226, 44-51.

Kohr G, Eckardt S, Luddens H, Monyer H, Seeburg PH. NMDA receptor channels: subunit-specific potentiation by reducing agents. Neuron. 1994; 12:1031-1040.

Lavoie, S., Murray, M.M., Deppen, P., Knyazeva, M.G., Berk, M., Boulat, O., Bovet, P., Bush, A.I., Conus, P., Copolov, D., et al. (2008). Glutathione precursor, N-acetyl-cysteine, improves mismatch negativity in schizophrenia patients. Neuropsychopharmacology: official publication of the American College of Neuropsychopharmacology 33, 2187-2199.

Lee, H., Dvorak, D., Kao, H.Y., Duffy, A.M., Scharfman, H.E., and Fenton, A.A. (2012). Early cognitive experience prevents adult deficits in a neurodevelopmental schizophrenia model. Neuron 75, 714-724.

Lewis, B.L., and O'Donnell, P. (2000). Ventral tegmental area afferents to the prefrontal cortex maintain membrane potential 'up' states in pyramidal neurons via D1 dopamine receptors. Cerebral cortex 10, 1168-1175.

Lewis, D.A., Curley, A.A., Glausier, J.R., and Volk, D.W. (2012). Cortical parvalbumin interneurons and cognitive dysfunction in schizophrenia. Trends in neurosciences 35, 57-67.

Light GA, Hsu JL, Hsieh MH, Meyer-Gomes K, Sprock J, Swerdlow NR, Braff DL. Gamma band oscillations reveal neural network cortical coherence dysfunction in schizophrenia patients. Biol Psychiatry. 2006; 60:1231-1240.

Lipska, B.K., Swerdlow, N.R., Geyer, M.A., Jaskiw, G.E., Braff, D.L., and Weinberger, D.R. (1995). Neonatal excitotoxic hippocampal damage in rats causes post-pubertal changes in prepulse inhibition of startle and its disruption by apomorphine. Psychopharmacol 132, 303-310.

Lynch ED, Kil J. Development of Ebselen, a Glutathione Peroxidase Mimic, for the Prevention and Treatment of Noise-Induced Hearing Loss. Semin Hear. 2009; 30:47-55.

Miller E, Mrowicka M, Saluk-Juszczak J, Ireneusz M. The Level of Isoprostanes as a Non-invasive Marker for in vivo Lipid Peroxidation in Secondary Progressive Multiple Sclerosis. Neurochem Res. 2011;36:1012-1016.

Moussawi, K., Pacchioni, A., Moran, M., Olive, M.F., Gass, J.T., Lavin, A., and Kalivas, P.W. (2009). N- Acetylcysteine reverses cocaine-induced metaplasticity. Nature neuroscience 12, 182-189.

Muller, A., Cadenas, E., Graf, P., and Sies, H. (1984). A novel biologically active seleno-organic compound--I. Glutathione peroxidase-like activity in vitro and antioxidant capacity of PZ 51 (Ebselen). Biochemical pharmacology 33, 3235-3239.

Muller A, Gabriel H, Sies H, Terlinden R, Fischer H, Romer A. A novel biologically active selenooorganic compound--VII. Biotransformation of ebselen in perfused rat liver. Biochem Pharmacol. 1988; 37:1103- 1109.

Nakazawa K, Zsiros V, Jiang Z, Nakao K, Kolata S, Zhang S, Belforte JE. GABAergic interneuron origin of schizophrenia pathophysiology. Neuropharmacology. 2012; 62:1574-1583.

Nehru B, Kanwar SS. Modulation by N-acetylcysteine of lead-induced alterations in rat brain: reduced glutathione levels and morphology. Toxicol Mech Methods. 2007;17:289-293.

Niwa, M., Kamiya, A., Murai, R., Kubo, K., Gruber, A.J., Tomita, K., Lu, L., Tomisato, S., Jaaro-Peled, H., Seshadri, S., et al. (2010). Knockdown of DISC1 by in utero gene transfer disturbs postnatal dopaminergic maturation in the frontal cortex and leads to adult behavioral deficits. Neuron 65, 480-489.

Noguchi N, Yoshida Y, Kaneda H, Yamamoto Y, Niki E. Action of ebselen as an antioxidant against lipid peroxidation. Biochem Pharmacol. 1992; 44:39-44.

Nwokocha, C.R., Baker, A., Douglas, D., McCalla, G., Nwokocha, M., and Brown, P.D. (2013). Apocynin ameliorates cadmium-induced hypertension through elevation of endothelium nitric oxide synthase. Cardiovascular toxicology 13, 357-363.

O'Donnell, P. (2011). Adolescent onset of cortical disinhibition in schizophrenia: insights from animal models. Schizophrenia bulletin 37, 484-492.

O'Donnell, P. (2012a). Cortical disinhibition in the neonatal ventral hippocampal lesion model of schizophrenia: New vistas on possible therapeutic approaches. Pharmacology & therapeutics 133, 19-25.

O'Donnell, P. (2012b). Cortical interneurons, immune factors and oxidative stress as early targets for schizophrenia. The European journal of neuroscience 35, 1866-1870.

O'Donnell, P. (2013). How can animal models be better utilized? In Schizophrenia: Evolution and Synthesis, S.M. Silverstein, B. Moghaddam, and T. Wykes, eds. (Cambridge, MA: MIT Press), pp. 205-216.

O'Donnell, P., Lewis, B.L., Weinberger, D.R., and Lipska, B.K. (2002). Neonatal hippocampal damage alters electrophysiological properties of prefrontal cortical neurons in adult rats. Cerebral cortex 12, 975-982.

Otte, D.M., Sommersberg, B., Kudin, A., Guerrero, C., Albayram, O., Filiou, M.D., Frisch, P., Yilmaz, O., Drews, E., Turck, C.W., et al. (2011). N-acetyl cysteine treatment rescues cognitive deficits induced by mitochondrial dysfunction in G72/G30 transgenic mice. Neuropsychopharmacology: official publication of the American College of Neuropsychopharmacology 36, 2233-2243.

Pawlas, N., and Malecki, A. (2007). Effects of ebselen on glutathione level in neurons exposed to arachidonic acid and 4-hydroxynonenal during simulated ischemia in vitro. Pharmacological reports: PR 59, 708-714.

Paxinos, G., and Watson, C. (1998). The rat brain in stereotaxic coordinates, Fourth Edition edn (San Diego: Academic Press).

Radi, R. (2004). Nitric oxide, oxidants, and protein tyrosine nitration. Proceedings of the National Academy of Sciences of the United States of America 101, 4003-4008.

Reiter R, Wendel A. Selenium and drug metabolism--II. Independence of glutathione peroxidase and reversibility of hepatic enzyme modulations in deficient mice. Biochem Pharmacol. 1984; 33:1923-1928.

Satoh T, Ishige K, Sagara Y. Protective effects on neuronal cells of mouse afforded by ebselen against oxidative stress at multiple steps. Neurosci Lett. 2004; 371:1-5.

Schmitz, C., and Hof, P.R. (2005). Design-based stereology in neuroscience. Neuroscience 130, 813-831.

Sies H. Ebselen: a glutathione peroxidase mimic. Methods Enzymol. 1994;234:476-482.

Spencer KM, Nestor PG, Perlmutter R, Niznikiewicz MA, Klump MC, Frumin M, Shenton ME, McCarley RW. Neural synchrony indexes disordered perception and cognition in schizophrenia. Proc Natl Acad Sci U S A. 2004; 101:17288-17293.

Steullet, P., Cabungcal, J.H., Kulak, A., Kraftsik, R., Chen, Y., Dalton, T.P., Cuenod, M., and Do, K.Q. (2010). Redox dysregulation affects the ventral but not dorsal hippocampus: impairment of parvalbumin neurons, gamma oscillations, and related behaviors. The Journal of neuroscience: the official journal of the Society for Neuroscience 30, 2547-2558.

Steullet, P., Neijt, H.C., Cuenod, M., and Do, K.Q. (2006). Synaptic plasticity impairment and hypofunction of NMDA receptors induced by glutathione deficit: relevance to schizophrenia. Neuroscience 137, 807-819.

Tosic, M., Ott, J., Barral, S., Bovet, P., Deppen, P., Gheorghita, F., Matthey, M.L., Parnas, J., Preisig, M., Saraga, M., et al. (2006). Schizophrenia and oxidative stress: glutamate cysteine ligase modifier as a susceptibility gene. Am J Hum Genet 79, 586-592.

Tseng, K.Y., Chambers, R.A., and Lipska, B.K. (2009). The neonatal ventral hippocampal lesion as a heuristic neurodevelopmental model of schizophrenia. Behavioural brain research 204, 295-305.

Tseng, K.Y., Lewis, B.L., Hashimoto, T., Sesack, S.R., Kloc, M., Lewis, D.A., and O'Donnell, P. (2008). A neonatal ventral hippocampal lesion causes functional deficits in adult prefrontal cortical interneurons. The Journal of neuroscience: the official journal of the Society for Neuroscience 28, 12691-12699.

Tseng, K.Y., and O'Donnell, P. (2007). D2 dopamine receptors recruit a GABA component for their attenuation of excitatory synaptic transmission in the adult rat prefrontal cortex. Synapse 61, 843-850.

Uhlhaas PJ, Singer W. Abnormal neural oscillations and synchrony in schizophrenia. Nat Rev Neurosci. 2010; 11:100-113.

Ullrich V, Weber P, Meisch F, von Appen F. Ebselen-binding equilibria between plasma and target proteins. Biochem Pharmacol. 1996; 52:15-19.

Umbricht, D., Schmid, L., Koller, R., Vollenweider, F.X., Hell, D., and Javitt, D.C. (2000). Ketamine-induced deficits in auditory and visual context-dependent processing in healthy volunteers: implications for models of cognitive deficits in schizophrenia. Archives of general psychiatry 57, 1139-1147.

Wendel A, Fausel M, Safayhi H, Tiegs G, Otter R. A novel biologically active seleno-organic compound--II. Activity of PZ 51 in relation to glutathione peroxidase. Biochem Pharmacol. 1984; 33:3241-3245.

Wijtenburg S A, Gaston FE, Spieker E A, Forenic S A, Kochunov P, Hong LE, Rowland L M. Reproducibility of phase rotation STEAM at 3T: Focus on glutathione. Magn Reson.Med. 2013 Oct 22. doi: 0.1002/mrm. 24959. [Epub ahead of print]

Wirth EK, Conrad M, Winterer J, Wozny C, Carlson BA, Roth S, Schmitz D, Bornkamm GW, Coppola V, Tessarollo L, Schomburg L, Kohrle J, Hatfield DL, Schweizer U. Neuronal selenoprotein expression is required for interneuron development and prevents seizures and neurodegeneration. FASEB J. 2010; 24:844-852.

Yao, J.K., and Keshavan, M.S. (2011). Antioxidants, redox signaling, and pathophysiology in schizophrenia: an integrative view. Antioxidants & redox signaling 15, 2011-2035.

Zhao R, Masayasu H, Holmgren A. Ebselen: a substrate for human thioredoxin reductase strongly stimulating its hydroperoxide reductase activity and a superfast thioredoxin oxidant. Proc Natl Acad Sci U S A. 2002; 99:8579-8584.

### Compounds

Representative compounds of the present invention are described throught the specification and claims.

A representative compound of glutathione peroxidase (GPx) mimics includes ebselen, (2-Phenyl-1,2-benzisoselenazol-3(2H)-one) with empirical formula C₁₃H₉NOSe, molecular weight 274.2 and a formula of:

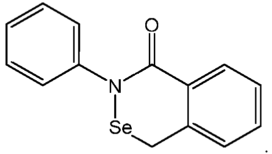

Ebselen is the only active ingredient administered in a formulation. Ebselen is slightly soluble in aqueous solutions at 25° Celsius. Ebselen acts as a catalyst and is not consumed during detoxification reactions (Muller et. al, 1988). An embodiment of an ebselen formulation is >99% pure as confirmed by HPLC. The synthesis of this formulation is provided by Rhodia Pharma Solutions and includes capsules that are hermetically sealed in blister packs. Each capsule contains 200 mg the ebselen formulation or SPI-1000 (placebo).

Other representative compounds of glutathione peroxidase (GPx) mimics include 2,2'-diseleno-bis-β-cyclodextrin and 6A,6B-diseleninic acid-6A',6B'-selenium bridged β-cyclodextrin.

Representative compounds of the second compound in the combination include glutathione (GSH), glutathione produgs listed in Table 1, and cysteine prodrugs listed in Table 2.

**Table 1 - glutathione prodrugs**

| STRUCTURE | NO. | NAME |
|---|---|---|
| 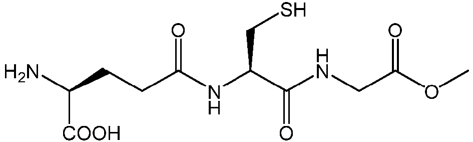 | 1 | *N⁵*-((*R*)-3-mercapto-1-((2-methoxy-2-oxoethyl)-amino)-1-oxopropan-2-yl)-L-glutamine |
| 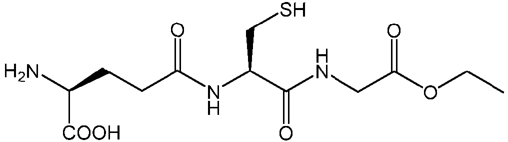 | 2 | *N⁵*-((*R*)-1-((2-ethoxy-2-oxoethyl)-amino)-3-mercapto-1-oxopropan-2-yl)-L-glutamine |
| 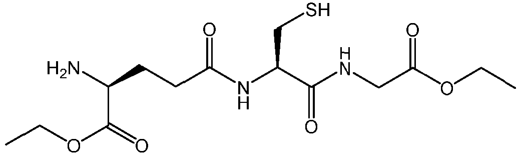 | 3 | ethyl *N⁵*-((*R*)-1-((2-ethoxy-2-oxoethyl)amino)-3-mercapto-1-oxopropan-2-yl)-L-glutaminate |
| 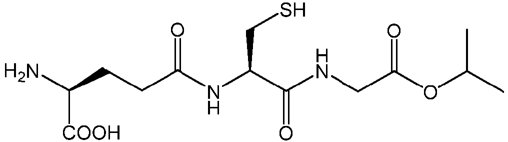 | 4 | *N⁵*-((*R*)-1-((2-isopropoxy-2-oxoethyl)amino)-3-mercapto-1-oxopropan-2-yl)-L-glutamine |
| 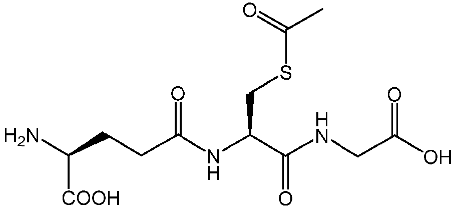 | 5 | *N⁵*-((*R*)-3-(acetylthio)-1-((carboxymethyl)amino)-1-oxopropan-2-yl)-L-glutamine |
| 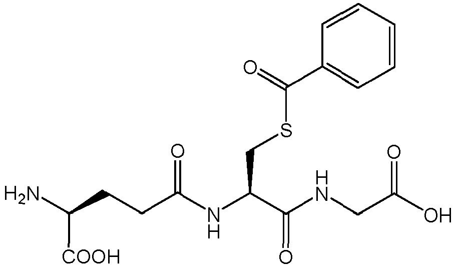 | 6 | *N⁵*-((*R*)-3-(benzoylthio)-1-((carboxymethyl)amino)-1-oxopropan-2-yl)-L-glutamine |
| 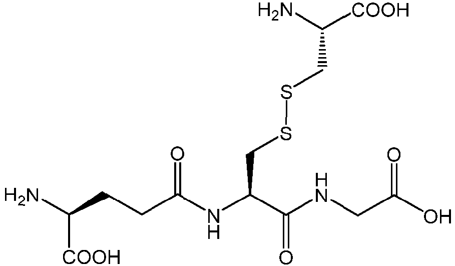 | 7 | *N⁵* -((*R*)-3-(((R)-2-amino-2-carboxyethyl)disulfanyl)-1-((carboxymethyl)amino)-1-oxopropan-2-yl)-L-glutamine |
| 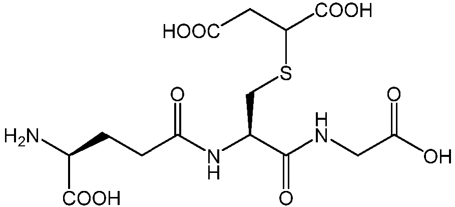 | 8 | 2-(((*R*)-2-((S)-4-amino-4-carboxybutanamido)-3-((carboxymethyl)amino)-3-oxopropyl)thio)succinic acid |

**Table 2 - cysteine prodrugs**

| STRUCTURE | NO. | NAME |
|---|---|---|
| 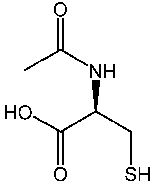 | 1 | *N*-acetyl cysteine |
| 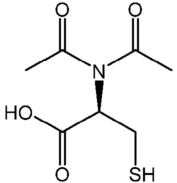 | 2 | *N*,*N*'-diacetyl cysteine |
| 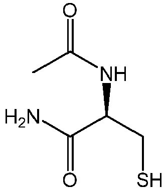 | 3 | *N*-acetyl cysteine amide |
| 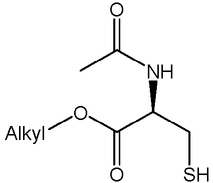 | 4 | *N*-acetyl cysteine alkyl esters |
| 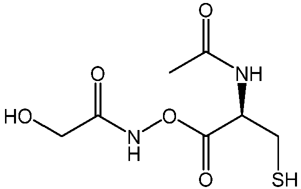 | 5 | *N*-acetyl cysteine glycolamide esters |
| 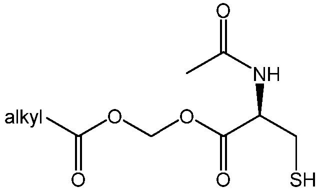 | 6 | *N*-acetyl cysteine acycloxymethyl esters |
| 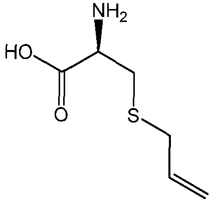 | 7 | S-allyl cysteine |
| 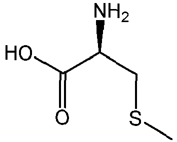 | 8 | S-methyl cysteine |
| 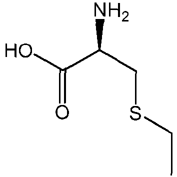 | 9 | S-ethyl cysteine |
| 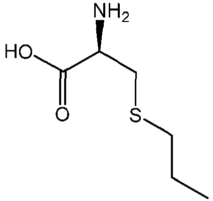 | 10 | S-propyl cysteine |
| 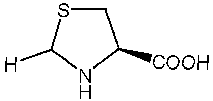 | 11 | (*R*)-thiazolidine-4-carboxylic acid |
| 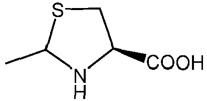 | 12 | (*4R*)-2-methylthiazolidine-4-carboxylic acid |
| 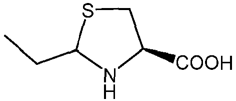 | 13 | (*4R*)-2-ethylthiazolidine-4-carboxylic acid |
| 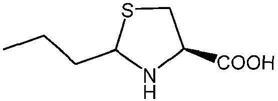 | 14 | (*4R*)-2-propylthiazolidine-4-carboxylic acid |
| 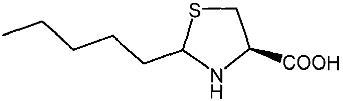 | 15 | (*4R*)-2-pentylthiazolidine-4-carboxylic acid |
| 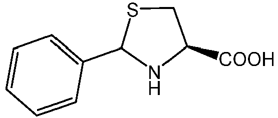 | 16 | (*4R*)-2-phenylthiazolidine-4-carboxylic acid |
| 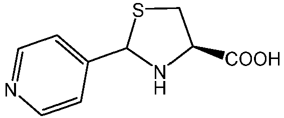 | 17 | (*4R*)-2-(pyridin-4-yl)thiazolidine-4-carboxylic acid |
| 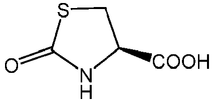 | 18 | (*R*)-2-oxothiazolidine-4-carboxylic acid |
| 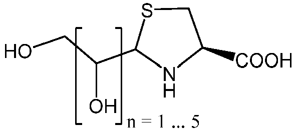 | 19 | For n = 3 (RibCys): |
| | | 2(*R,S*)-D-ribo-(1',2',3',4'-tetrahydroxybutyl)-thiazolidine-4(*R*)-carboxylic acid |
| 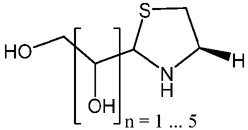 | 20 | For n = 3 (RibCyst): |
| | | 2(*R*,*S*)-D-ribo-(1',2',3',4'-tetrahydroxybutyl)-thiazolidine |

Some other embodiments of cysteine prodrugs include 2-substituted thiazolidine-4-carboxylic acids with aldose monosaccarides, such as glyceraldehyde, arabinose, lyxose, ribose, xylose, galactose, glucose, and mannose.

Where the compounds according to this invention have at least one chiral center, they may accordingly exist as enantiomers. Where the compounds possess two or more chiral centers, they may additionally exist as diastereomers. Where the processes for the preparation of the compounds according to the invention give rise to mixtures of stereoisomers, these isomers may be separated by conventional techniques such as preparative chromatography. The compounds may be prepared in racemic form or as individual enantiomers or diasteromers by either stereospecific synthesis or by resolution. The compounds may, for example, be resolved into their component enantiomers or diastereomers by standard techniques, such as the formation of stereoisomeric pairs by salt formation with an optically active base, followed by fractional crystallization and regeneration of the free acid. The compounds may also be resolved by formation of stereoisomeric esters or amides, followed by chromatographic separation and removal of the chiral auxiliary. Alternatively, the compounds may be resolved using a chiral HPLC column. It is to be understood that all stereoisomers, racemic mixtures, diastereomers, geometric isomers, and enantiomers thereof are encompassed within the scope of the present invention.

Furthermore, some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

### General Administration, Formulation, and Dosages

The present compounds are GPx modulators and are therefore useful in treating, preventing, or inhibiting the progression of GPx mediated conditions including but not limited to schizophrenia, bipolar disorder, psychotic disorders, and other disorders, diseases, or conditions related thereto.

An embodiment features a method for treating a subject with a GPx mediated disease, said method comprising administering to the subject a therapeutically effective amount of a pharmaceutical composition comprising a compound disclosed herein. In particular, the embodiment also provides a method for treating or inhibiting the progression of schizophrenia, bipolar disorder, psychotic disorders, tardive dyskinesia, and associated symptoms or complications thereof in a subject, wherein the method comprises administering to the subject a therapeutically effective amount of a pharmaceutical composition comprising a compound disclosed herein.

Embodiments also include prodrugs of the compounds disclosed herein. In general, such prodrugs will be functional derivatives of the compounds which are readily convertible *in vivo* into the required compound. Thus, in the methods of treatment of the present invention, the term "administering" shall encompass the treatment of the various disorders described with the compound specifically disclosed or with a compound which may not be specifically disclosed, but which converts to the specified compound *in vivo* after administration to the subject. Conventional procedures for the selection and preparation of suitable prodrug derivatives are described, for example, in "Design of Prodrugs", ed. H. Bundgaard, Elsevier, 1985.

Some of the crystalline forms for the compounds may exist as polymorphs and as such are intended to be included in the present invention. In addition, some of the compounds may form solvates with water (i.e., hydrates) or common organic solvents, and such solvates are intended to be encompassed by some embodiments.

Where the processes for the preparation of the compounds as disclosed herein give rise to mixtures of stereoisomers, these isomers may be separated by conventional techniques such as preparative chromatography. The compounds may be prepared in racemic form or as individual enantiomers or diasteromers by either stereospecific synthesis or by resolution. The compounds may, for example, be resolved into their component enantiomers or diastereomers by standard techniques, such as the formation of stereoisomeric pairs by salt formation with an optically active base, followed by fractional crystallization and regeneration of the free acid. The compounds may also be resolved by formation of stereoisomeric esters or amides, followed by chromatographic separation and removal of the chiral auxiliary. Alternatively, the compounds may be resolved using a chiral HPLC column. It is to be understood that all stereoisomers, racemic mixtures, diastereomers, cis-trans isomers, and enantiomers thereof are encompassed by some embodiments.

### Dosages

Those of skill in the treatment of disorders, diseases, or conditions mediated by GPx can determine the effective daily amount from the test results presented hereinafter and other information. The exact dosage and frequency of administration depends on the particular compound of invention used, the particular condition being treated, the severity of the condition being treated, the age, weight and general physical condition of the particular patient as well as other medication the patient may be taking, as is well known to those skilled in the art. Furthermore, it is evident that said effective daily amount may be lowered or increased depending on the response of the treated patient and/or depending on the evaluation of the physician prescribing the compounds of the instant invention. The effective daily amount ranges mentioned herein are therefore only guidelines in practicing the present invention.

For the methods for the treatment of GPx mediated disorders described herein using any of the compounds as disclosed herein, the dosage form will contain a pharmaceutically acceptable carrier containing between from about 0.1 mg to about 5000 mg; particularly from about 0.5 mg to about 1000 mg; and, more particularly, from about 1 mg to about 100 mg of the compound, and may be constituted into any form suitable for the mode of administration selected. The dosages, however, may be varied depending upon the requirement of the subjects, the severity of the condition being treated and the compound being employed. The use of either daily administration or post-periodic dosing may be employed.

The pharmaceutical compositions herein will contain, per unit dosage unit, e.g., tablet, capsule, powder, injection, suppository, teaspoonful and the like, of from about 0.001 mg/kg/day to about 10 mg/kg/day (particularly from about 0.01 mg/kg/day to about 1 mg/kg/day; and, more particularly, from about 0.1 mg/kg/day to about 0.5 mg/kg/day) and may be given at a dosage of from about 0.001 mg/kg/day to about 30 mg/kg/day (particularly from about 0.01 mg/kg/day to about 2 mg/kg/day, more particularly from about 0.1 mg/kg/day to about 1 mg/kg/day and even more particularly from about 0.5 mg/kg/day to about 1 mg/kg/day).

These compositions are in unit dosage forms from such as tablets, pills, capsules, dry powders for reconstitution or inhalation, granules, lozenges, sterile parenteral solutions or suspensions, metered aerosol or liquid sprays, drops, ampoules, autoinjector devices or suppositories for administration by oral, intranasal, sublingual, intraocular, transdermal, parenteral, rectal, vaginal, dry powder inhaler or other inhalation or insufflation means. Alternatively, the composition may be presented in a form suitable for once-weekly or once-monthly administration; for example, an insoluble salt of the active compound, such as the decanoate salt, may be adapted to provide a depot preparation for intramuscular injection.

For preparing solid pharmaceutical compositions such as tablets, the principal active ingredient is mixed with a pharmaceutical carrier, e.g. conventional tableting ingredients such as diluents, binders, adhesives, disintegrants, lubricants, antiadherents and gildants. Suitable diluents include, but are not limited to, starch (i.e. corn, wheat, or potato starch, which may be hydrolized), lactose (granulated, spray dried or anhydrous), sucrose, sucrose-based diluents (confectioner's sugar; sucrose plus about 7 to 10 weight percent invert sugar; sucrose plus about 3 weight percent modified dextrins; sucrose plus invert sugar, about 4 weight percent invert sugar, about 0.1 to 0.2 weight percent cornstarch and magnesium stearate), dextrose, inositol, mannitol, sorbitol, microcrystalline cellulose (i.e. AVICEL^{™} microcrystalline cellulose available from FMC Corp.), dicalcium phosphate, calcium sulfate dihydrate, calcium lactate trihydrate and the like. Suitable binders and adhesives include, but are not limited to acacia gum, guar gum, tragacanth gum, sucrose, gelatin, glucose, starch, and cellulosics (i.e. methylcellulose, sodium carboxymethylcellulose, ethylcellulose, hydroxypropylmethylcellulose, hydroxypropylcellulose, and the like), water soluble or dispersible binders (i.e. alginic acid and salts thereof, magnesium aluminum silicate, hydroxyethylcellulose [i.e. TYLOSE^{™} available from Hoechst Celanese], polyethylene glycol, polysaccharide acids, bentonites, polyvinylpyrrolidone, polymethacrylates and pregelatinized starch) and the like. Suitable disintegrants include, but are not limited to, starches (corn, potato, etc.), sodium starch glycolates, pregelatinized starches, clays (magnesium aluminum silicate), celluloses (such as crosslinked sodium carboxymethylcellulose and microcrystalline cellulose), alginates, pregelatinized starches (i.e. corn starch, etc.), gums (i.e. agar, guar, locust bean, karaya, pectin, and tragacanth gum), cross-linked polyvinylpyrrolidone and the like. Suitable lubricants and antiadherents include, but are not limited to, stearates (magnesium, calcium and sodium), stearic acid, talc waxes, stearowet, boric acid, sodium chloride, DL-leucine, carbowax 4000, carbowax 6000, sodium oleate, sodium benzoate, sodium acetate, sodium lauryl sulfate, magnesium lauryl sulfate and the like. Suitable gildants include, but are not limited to, talc, cornstarch, silica (i.e. CAB-O-SIL^{™} silica available from Cabot, SYLOID^{™} silica available from W. R. Grace/Davison, and AEROSIL^{™} silica available from Degussa) and the like. Sweeteners and flavorants may be added to chewable solid dosage forms to improve the palatability of the oral dosage form. Additionally, colorants and coatings may be added or applied to the solid dosage form for ease of identification of the drug or for aesthetic purposes. These carriers are formulated with the pharmaceutical active to provide an accurate, appropriate dose of the pharmaceutical active with a therapeutic release profile.

Generally these carriers are mixed with the pharmaceutical active to form a solid preformulation composition containing a homogeneous mixture of the pharmaceutical active form of the present invention, or a pharmaceutically acceptable salt thereof. Generally the preformulation will be formed by one of three common methods: (a) wet granulation, (b) dry granulation and (c) dry blending. When referring to these preformulation compositions as homogeneous, it is meant that the active ingredient is dispersed evenly throughout the composition so that the composition may be readily subdivided into equally effective dosage forms such as tablets, pills and capsules. This solid preformulation composition is then subdivided into unit dosage forms of the type described above containing from about 0.1 mg to about 500 mg of the active ingredient of the present invention. The tablets or pills containing the novel compositions may also be formulated in multilayer tablets or pills to provide a sustained or provide dual-release products. For example, a dual release tablet or pill can comprise an inner dosage and an outer dosage component, the latter being in the form of an envelope over the former. The two components can be separated by an enteric layer, which serves to resist disintegration in the stomach and permits the inner component to pass intact into the duodenum or to be delayed in release. A variety of materials can be used for such enteric layers or coatings, such materials including a number of polymeric materials such as shellac, cellulose acetate (i.e. cellulose acetate phthalate, cellulose acetate trimetllitate), polyvinyl acetate phthalate, hydroxypropyl methylcellulose phthalate, hydroxypropyl methylcellulose acetate succinate, methacrylate and ethylacrylate copolymers, methacrylate and methyl methacrylate copolymers and the like. Sustained release tablets may also be made by film coating or wet granulation using slightly soluble or insoluble substances in solution (which for a wet granulation acts as the binding agents) or low melting solids a molten form (which in a wet granulation may incorporate the active ingredient). These materials include natural and synthetic polymers waxes, hydrogenated oils, fatty acids and alcohols (i.e. beeswax, carnauba wax, cetyl alcohol, cetylstearyl alcohol, and the like), esters of fatty acids metallic soaps, and other acceptable materials that can be used to granulate, coat, entrap or otherwise limit the solubility of an active ingredient to achieve a prolonged or sustained release product.

The liquid forms in which the novel compositions disclosed herein may be incorporated for administration orally or by injection include, but are not limited to aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles. Suitable suspending agents for aqueous suspensions, include synthetic and natural gums such as, acacia, agar, alginate (i.e. propylene alginate, sodium alginate and the like), guar, karaya, locust bean, pectin, tragacanth, and xanthan gum, cellulosics such as sodium carboxymethylcellulose, methylcellulose, hydroxymethylcellulose, hydroxyethylcellulose, hydroxypropyl cellulose and hydroxypropyl methylcellulose, and combinations thereof, synthetic polymers such as polyvinyl pyrrolidone, carbomer (i.e. carboxypolymethylene), and polyethylene glycol; clays such as bentonite, hectorite, attapulgite or sepiolite; and other pharmaceutically acceptable suspending agents such as lecithin, gelatin or the like. Suitable surfactants include but are not limited to sodium docusate, sodium lauryl sulfate, polysorbate, octoxynol-9, nonoxynol-10, polysorbate 20, polysorbate 40, polysorbate 60, polysorbate 80, polyoxamer 188, polyoxamer 235 and combinations thereof. Suitable deflocculating or dispersing agent include pharmaceutical grade lecithins. Suitable flocculating agent include but are not limited to simple neutral electrolytes (i.e. sodium chloride, potassium, chloride, and the like), highly charged insoluble polymers and polyelectrolyte species, water soluble divalent or trivalent ions (i.e. calcium salts, alums or sulfates, citrates and phosphates (which can be used jointly in formulations as pH buffers and flocculating agents). Suitable preservatives include but are not limited to parabens (i.e. methyl, ethyl, n-propyl and n-butyl), sorbic acid, thimerosal, quaternary ammonium salts, benzyl alcohol, benzoic acid, chlorhexidine gluconate, phenylethanol and the like. There are many liquid vehicles that may be used in liquid pharmaceutical dosage forms, however, the liquid vehicle that is used in a particular dosage form must be compatible with the suspending agent(s). For example, nonpolar liquid vehicles such as fatty esters and oils liquid vehicles are best used with suspending agents such as low HLB (Hydrophile-Lipophile Balance) surfactants, stearalkonium hectorite, water insoluble resins, water insoluble film forming polymers and the like. Conversely, polar liquids such as water, alcohols, polyols and glycols are best used with suspending agents such as higher HLB surfactants, clays silicates, gums, water soluble cellulosics, water soluble polymers and the like. For parenteral administration, sterile suspensions and solutions are desired. Liquid forms useful for parenteral administration include sterile solutions, emulsions and suspensions. Isotonic preparations which generally contain suitable preservatives are employed when intravenous administration is desired.

Furthermore, compounds disclosed herein can be administered in an intranasal dosage form via topical use of suitable intranasal vehicles or via transdermal skin patches, the composition of which are well known to those of ordinary skill in that art. To be administered in the form of a transdermal delivery system, the administration of a therapeutic dose will, of course, be continuous rather than intermittent throughout the dosage regimen.

Compounds disclosed herein can also be administered in the form of liposome delivery systems, such as small unilamellar vesicles, large unilamellar vesicles, multilamellar vesicles and the like. Liposomes can be formed from a variety of phospholipids, such as cholesterol, stearylamine, phosphatidylcholines and the like.

The daily dose of a pharmaceutical composition disclosed herein may be varied over a wide range from about 0.1 mg to about 5000 mg; preferably, the dose will be in the range of from about 1 mg to about 100 mg per day for an average human. For oral administration, the compositions are preferably provided in the form of tablets containing, 0.01, 0.05, 0.1, 0.5, 1.0, 2.5, 5.0, 10.0, 15.0, 25.0, 50.0, 100, 150, 200, 250 or 500 milligrams of the active ingredient for the symptomatic adjustment of the dosage to the subject to be treated. Advantageously, a compound of the present invention may be administered in a single daily dose or the total daily dosage may be administered in divided doses of two, three or four times daily.

The therapeutically effective dose for active compounds disclosed herein or a pharmaceutical composition thereof may vary according to the desired effect. Therefore, optimal dosages to be administered may be readily determined by those skilled in the art, and may vary with the particular compound used, the mode of administration, the strength of the preparation, and the advancement of the disease condition. In addition, factors associated with the particular subject being treated, including subject age, weight, diet and time of administration, will result in the need to adjust the dose to an appropriate therapeutic level. The above dosages are thus exemplary of the average case. There can, of course, be individual instances where higher or lower dosage ranges are merited, and such are within the scope of this invention.

Compounds disclosed herein may be administered in any of the foregoing compositions and dosage regimens or by means of those compositions and dosage regimens established in the art whenever use of the compounds disclosed herein as GPx modulators is required for a subject in need thereof.

### Formulations

To prepare the pharmaceutical compositions disclosed herein, one or more compounds disclosed herein or salt thereof as the active ingredient, is intimately admixed with a pharmaceutical carrier according to conventional pharmaceutical compounding techniques, which carrier may take a wide variety of forms depending of the form of preparation desired for administration (e.g. oral or parenteral). Suitable pharmaceutically acceptable carriers are well known in the art. Descriptions of some of these pharmaceutically acceptable carriers may be found in The Handbook of Pharmaceutical Excipients, published by the American Pharmaceutical Association and the Pharmaceutical Society of Great Britain.

The compounds of the present invention may be formulated into various pharmaceutical forms for administration purposes. Methods of formulating pharmaceutical compositions have been described in numerous publications such as Pharmaceutical Dosage Forms: Tablets, Second Edition, Revised and Expanded, Volumes 1-3, edited by Lieberman et al; Pharmaceutical Dosage Forms: Parenteral Medications, Volumes 1-2, edited by Avis et al; and Pharmaceutical Dosage Forms: Disperse Systems, Volumes 1-2, edited by Lieberman et al; published by Marcel Dekker, Inc.

An ebselen formulation in form of capsule was prepared for below examples that investigated ebselen as a GPx modulator, where GPx, an enzyme dysregulated in schizophrenia (SZ) patients, is a novel therapeutic SZ target. Ebselen is the only active ingredient in that formulation, acting as a catalyst and not being consumed during detoxification reactions. The formulation is >99% pure as determined by HPLC. The capsules are hermetically sealed in blister packs. Each capsule contains 200 mg of ebselen that possesses a low toxicity because of its unique structure stability. Its selenium (Se) moiety is not liberated during biotransformation and therefore does not enter selenium metabolism. It is possible that in the process of manufacture, there will be remaining unbound selenium present. The manufacturing criterion is that each capsule contains less than 1 microgram of inorganic selenium. In humans, selenium toxicity, or selenosis, can occur following chronic ingestion of high quantities of selenium. The Recommended Daily Allowance (RDA) of selenium for adults is 55 microgram per day. Dosage is adjusted to result in the total selenium exposure being significantly less than RDA which is monitored during the study.

### Methods of Use

Also disclosed herein are methods of using neonatal ventral hippocampal lesion (NVHL) rats and methods related to analyzing/diagnosing animal models representative of GPx mediated disorders, including but not limited to oxidative stress, schizophrenia, bipolar disorder and other psychotic disorders. Uses of these methods disclosed herein can include research applications, therapeutic purposes, medical diagnostics, and/or stratifying one or more patients or subjects. Methods of identifying compositions that are useful for the prevention or treatment of GPx mediated disorders are disclosed.

Some embodiments of these methods emphasize early phase evaluation of the ebselen mechanisms of action as disclosed herein and brain biomarker engagement in schizophrenia patients. One embodiment uses corroborative brain magnetic resonance spectroscopy of GSH and other peripheral blood biomarker of GSH as primary outcomes, e.g. in a clinical trial.

### Methods of Treating Oxidative Stress, Schizophrenia and other Psychotic Diseases

Increased level of ***oxidative stress*** immunolabeling in the PFC of juvenile NVHL rats was observed along with a decrease in PV cell counts, PPI deficit, altered dopamine modulation of local PFC circuits, and deficits in evoked-related potentials in the EEG of adult NVHL rats. All these deficits were prevented with ***N-acetyl cysteine*** (NAC) treatment from P5 to P50. PPI deficits were also prevented if NAC treatment was initiated during adolescence (P35) and by two other redox modulators ***(ebselen, Apocynin*).** The data showed that oxidative stress in prefrontal cortex is a core feature mediating alterations induced by the NVHL, and antioxidant treatment prevents these alterations. Presymptomatic oxidative stress, highly present in PVI and also observed in pyramidal neurons, is therefore responsible for diverse schizophrenia-relevant phenomena in a neurodevelopmental model that does not entail a direct manipulation of redox pathways.

Oxidative stress can affect PFC function via several mechanisms. With high levels of oxidative stress, cell damage or death can occur via membrane lipid peroxidation, DNA mutagenesis, alterations in chromatin structure, inactivation of critical enzymes, or activation of kinase and caspase cascades (Bitanihirwe and Woo, 2011). Redox imbalance can also lead to brain development disturbances by affecting redox-sensitive cysteine residues at the DNA-binding sites of transcription factors (Haddad, 2002) and affecting mitochondrial DNA, highly susceptible to oxidation (Jones and Go, 2010).

Furthermore, many synaptic proteins include regulatory redox sites; for example, NMDA receptors become hypofunctional following oxidation (Steullet et al., 2006). Oxidative stress and nitrosative stress was detected in PFC pyramidal neurons and PVI in juvenile rats with a NVHL prior to the onset of electrophysiological and behavioral deficits. This indicates PVI may still be somewhat functional, and that upon their periadolescent maturation the deleterious effect of oxidative stress renders them into a diseased state as revealed by the reduction in PV and PNN labeling. Our data indicate that redox alterations in the NVHL model encompass both oxidative and nitrosative stress, and treatments that increase GHS (NAC and ebselen) or decrease reactive oxygen species (ROS) generation (apocynin) prevent adult-onset behavioral deficits. Thus, the NVHL model presents a widespread alteration in redox pathways that could be reversed by targeting different modulators, such as GSH and NADPH oxidase.

Oxidative stress is also seen in another animal model of schizophrenia: the dominant negative DISC1 (DN-DISC1) mouse (Johnson et al., 2013). DN-DISC1 mice have increased 8-oxo-dG staining in the PFC that is associated with several behavioral deficits. The data indicates a causal link between heightened oxidative stress in the PFC and the electrophysiological and behavioral deficits associated with schizophrenia, as the anti-oxidant NAC prevents both the increase in oxidative stress and electrophysiological and behavioral deficits in NVHL rats.

PFC physiology was dysfunctional in adult NVHL rats, and this deficit was prevented by NAC treatment. Several endpoints were used to assess PFC function, including dopamine modulation of synaptic responses in pyramidal neurons in slices, in vivo intracellular recordings of responses to VTA stimulation, and auditory evoked potentials. The recordings from pyramidal neurons showed loss of D2-mediated attenuation of cortico-cortical EPSPs in slices and exaggerated firing evoked by VTA stimulation in vivo in adult NVHL rats, also reported by O'Donnell et al., 2002; Tseng et al., 2008. Both the slice D2 attenuation of pyramidal cell synaptic responses and the in vivo silencing of pyramidal neurons by VTA stimulation are dependent on activation of FSI by dopamine in naïve rats (Tseng and O'Donnell, 2007). The absence of alterations in these responses in NAC-treated NVHL rats indicates that oxidative stress during postnatal development has a deleterious effect on dopamine-modulated FSI-pyramidal cell interactions. These interactions are critical for proper excitation-inhibition balance and information processing in the PFC. Currently, there is a debate as to whether interneurons or pyramidal neurons are the primary site of dysfunction in schizophrenia. Our data are agnostic to which cell type is primarily affected and highlights oxidative stress as a cause of altered interactions between pyramidal neurons and inhibitory interneurons.

Mismatch negativity is a measure of high translational relevance. MMN tests the attribution of saliency to deviant auditory stimuli, and it is disrupted in schizophrenia patients (Javitt et al., 1993). As MMN is dependent on NMDA receptor activity (Ehrlichman et al., 2009), it is likely that oxidative stress impairs NMDA-dependent synaptic cortical mechanisms involved in processing of salient vs. common signals.

MMN deficits in NVHL rats were observed, which were prevented by NAC treatment. The functional assessment of the impact of antioxidant treatment was complemented by testing of sensorimotor integration with PPI. Both juvenile and adolescent-only NAC treatment prevented adult PPI deficits in NVHL rats. The observation that adolescent treatment with NAC or Ebselen is sufficient to prevent PPI deficits has important implications for redox mechanisms as potential targets for schizophrenia treatment. A deficit is likely prevented even if antioxidant treatment is initiated after development of oxidative stress. As ultra-high risk subjects for schizophrenia cannot be identified until adolescence, redox modulation can be beneficial even if initiated once high risk has been identified.

Presymptomatic oxidative stress was shown to likely cause aberrant adult PFC function in NVHL rats. This developmental manipulation is a well-established model of altered cortical excitation-inhibition balance. Although the model entails a lesion, which is not observed in schizophrenia, the NVHL and other developmental models have been useful to test specific hypotheses about developmental trajectories of electrophysiological and behavioral phenomena of relevance to the disease (O'Donnell, 2013). Major strengths of the NVHL model include the adolescent onset of deficits and the ability to reproduce phenomena observed in schizophrenia when translatable measures are evaluated (O'Donnell, 2012a). Remarkably, the NVHL model converges with several other manipulations deemed as animal models of schizophrenia in producing loss of PVI immunolabeling and altered excitation-inhibition balance (O'Donnell, 2011).

Despite their limitations, the behavioral and physiological endpoints used in the disclosed experiments are widely used to assess integrity of cortical inhibitory networks and their impact on pyramidal cell activity. Inhibitory networks, developing at the time of the lesion and beyond, play a crucial role in experience-dependent refinement of neural networks (Hensch, 2005) that extends into adolescence. This role may be reflected in cognitive training during adolescence preventing cognitive impairments in adult NVHL rats (Lee et al., 2012) and adolescent stress unmasking latent neuropathology in mice with maternal immune activation (Giovanoli et al., 2013). Adolescence is therefore a critical developmental stage in which pathophysiological conditions involving oxidative stress can affect a still developing PFC, but it yet provides a window of opportunity for therapeutic intervention. This suggests that antioxidants or redox regulators without serious side effects may prove effective to reduce conversion in subjects at risk for psychiatric disorders by preventing pathophysiological changes associated with loss of cortical PVI function.

### EXAMPLES

Below are examples of specific embodiments for carrying out the present invention. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

The practice of the present invention will employ, unless otherwise indicated, conventional methods of protein chemistry, biochemistry, recombinant DNA techniques and pharmacology, within the skill of the art. Such techniques are explained fully in the literature. See, e.g., T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 18th Edition (Easton, Pennsylvania: Mack Publishing Company, 1990); Carey and Sundberg Advanced Organic Chemistry 3rd Ed. (Plenum Press) Vols A and B(1992).

### Example 1: Brain GSH, peripheral GSH and lipid pexoxidation markers

This example describes the physiological effects on a patient who was treated with ebselen alone or in combination with another antipsychotic drug. One of the primary outcomes of ebselen in preclinical studies showed the elevation of neuronal GSH levels, which were previously shown to be reduced for schizophrenic (SZ) patients. (Do et al., 2000; Wood et al. 2009). Magnetic resonance spectroscopy (MRS) was refined to monitor GSH during brain target engagement by ebselen. To assess the effect size using a magnetic resonance scanner, very short echo time 1H-MRS sequence was used to assess GSH levels in 11 schizophrenic patients and 11 normal control patients. GSH at the anterior cingulate cortex (ACC) as shown in FIGS. 2A and 2B was reduced in SZ patients compared with normal control patients (mean±sd = 2.4±0.3 vs. 2.6±0.4, F=2.0, p=0.17; d=0.65). Using this method spectral quantification yielded excellent fits for GSH and other components with Cramer-Rao lower bounds (CRLB) less than 10.

MRS GSH test-retest reproducibility for spectroscopic voxel was assessed one week apart in five participants. The reproducibility was excellent (CV range: 1.0-2.3; GSH=1.0; percent difference range: 1.5-4.2%; GSH=1.5%) with intraclass correlation ICC=0.967. To determine the accuracy in quantifying GSH, five phantoms with increasing concentrations of GSH (0 mM, 1.0 mM, 2.5 mM, 5 mM, and 10 mM) were fabricated. To test the specificity of separating GSH from other metabolites, each phantom was also mixed with myo-inositol, creatine, glucose, glutamate, glutamine, and y-aminobuytric acid. A linear regression was computed to determine fit to the standard curve, which yielded excellent precision and accuracy in detecting glutathione concentration, where the LC Model quantified phantom GSH concentration corresponded to the known GSH concentration at r² = 0.994. (Wijtenburg et al., 2013). To address the macromolecule background, metabolite nulled (i.e. macromolecule spectra) is acquired for each participant to be used for each participant's spectral fitting. Beside group comparisons with placebo, ebselen dose-dependent and plasma level-dependent brain GSH changes by ebselen can be tested.

Peripheral GSH, GSSG, and GPx provide another way for monitoring the mechanisms by which elselen engages the redox pathway. Although direct comparisons of blood vs. brain tissue GSH/GSSG/GPx have not been made, peripheral glutathione administration increases brain GSH level. (Nehru et al., 2007). Ebselen effects can be transmitted through GSH or also GSSG and GPx changes. To examine if ebselen exerts its effect through reducing lipid peroxidation, one can measure isoprostanes, a marker of lipid peroxidation that is formed by peroxidation of membrane phospholipids. Urinary isoprostane is elevated in some white matter diseases (Miller et al., 2011) and in SZ patients (Dietrich-Muszalska et al., 2009). One can use isoprostane to index potential effect on reducing lipid peroxidation by elselen.

### Example 2: Electrophysiology biomarkers mismatch negativity and gamma oscillations

This example describes mismatch negativity (MMN) for N-methyl-D-asparatate receptors that are redox-sensitive proteins contained in redox modulatory sites (Do et al., 2009, Gonzalez-Burgos et al., 2012, Choi et al., 2012, Kohr, et al., 1994, Nakazawa, et al., 2012). Ebselen modulates this redox modulatory site and increases neuron viability. (Herin, et al., 2001). Schizophrenia (SZ) is associated with N-methyl-D-asparatate receptors dysfunction, thought to be indexed by mismatch negativity (Javitt, et al., 1993, Javitt, et al., 1998, Javitt, et al., 1996). Administration of n-acetylcysteine improved mismatch negativity in SZ (Berk, et al., 2008, Lavoie, et al., 2008). MMN and peripheral GSH were significantly correlated in controls as shown in FIG. 3. The correlation was not significant in SZ plausibly due to dysregulated relationships, since both MMN and GSH are reduced in SZ as illustrated in FIGS. 3A-3C. MMN may serve as a biomarker to test whether ebselen effects, if present, are mediated through a NMDAR-related mechanism.

Gamma frequency neural oscillations: Parvalbumin (PV) interneurons, critical cells for the generation of gamma oscillations (Carlen, et al., 2012), are another cellular component sensitive to oxidative stress (Do, et al., 2009, Gonzalez-Burgos et al., 2012, Choi et al., 2012, Kohr, et al., 1994, Nakazawa, et al., 2012). Gamma band and PV-interneuron abnormalites are frequently reported in SZ (Spencer, et al., 2004, Light, et al., 2006, Cho, et al., 2006, Uhlhaas, et al., 2010, Hong, et al., 2004). GPx mutant mice showed specific reduction in PV-positive interneurons (Wirth, 2010). Animals with low GSH showed pronounced beta and gamma oscillation deficits due to impaired PV interneuron function (Steullet, et al., 2010). Gamma band at 21-40 and 41-85 Hz were reduced in SZ compared with normal control patients (all p<0.001) and correlated with GSH (r=0.40-0.59, p=0.042-0.001). Multivariate mediation modeling showed that gamma response at 20-40 Hz was a significant mediator for the GSH effect on function capacity as measured by UPSA-2 as illustrated in FIG. 4 (Ballesteros, et al., 2013b). Gamma oscillations may be used as an alternative biomarker for indication on whether ebselen improves clinical outcome, if present, by improving PV interneuron function.

Some embodiments of the diagnostic methods aim to identify biomarkers that index target engagement by ebselen, and provides empirical evidence for biomarker selection for later trials; or in case of a non-significant trial, indicate where target engagement might have failed.

### Example 3: Glutathione peroxidase (GPx) mimics - mechanism of action.

Ebselen is a small molecule mimic of GPx, which in humans entails a family of 8 isozymes with similar peroxidase functions. Most GPx isozymes reduce reactive oxygen/nitrogen species (ROS/RNS) by binding of free radicals to its selenium (Se) moiety. By reacting with GSH, GPx is cytoprotective by limiting free radical toxicity through reducing hydrogen peroxide (H₂O₂) (FIG. 5A, Wendel, et al., 1984, Reiter, et al., 1984, Muller, et al., 1984), peroxynitrite (ONOO⁻), a RNS radical formed by two free radicals, super oxide anion and nitric oxide (FIG. 5B, Noguchi, et al., 1992, Daiber, et al., 2000), and lipid hydroperoxide (LOOH) to redox-inert alcohols (FIG. 5C, Sies, 1994). Ebselen is also a substrate of theoredoxin (Trx) as shown in FIG. 5D (Zhao, et al., 2002). However, administration of GPx enzyme itself is not practical due to its large size and instability.

As illustrated in FIGS. 5, the GPx mimic ebselen enhances the redox cycle of GSH→GSSG→GSH, thus recycling GSH. This catalytic mechanism is different from NAC's mechanism of action. NAC contains cysteine with a sulfhydryl group that acts as an antioxidant. However, NAC does not preserve GSH levels as efficiently as ebselen and does not induce GPx activity that helps recycling GSH. NAC supplies an amino acid: cysteine. Like any amino acid, cysteine serves diverse functions. Clearly, more work is required to support or refute whether NAC and/or ebselen is a better choice for SZ. In comparison, ebselen has a well-defined mechanism of action.

Animal experiments are consistent in showing that ebselen increases GSH and replenishes GSH depleted by neurotoxic mechanisms as illustrated in FIGS. 6. This enhanced GPx activity may also spare the endogenously generated GSH in a disease where oxidative stress is increased (Do, et al., 2009, Kano, et al., 2013) and GSH synthesis is decreased (Gysin, et al., 2007), resulting in higher availability of GSH. Ebselen's efficient effect on GSH is evident by multiple pre-clinical studies consistently showing that treatment with ebselen dose-dependently increases GSH levels in neurons, astrocytes and other cell types.

FIGS. 6 further illustrate the dose-dependent rise of GSH by ebselen was observed in both basal and stressed conditions in neurons (Pawlas, et al., 2007). Increased glutamate can be neurotoxic and deplete GSH; ebselen itself increased GSH, and combining glutamate with ebselen neutralized the GSH depletion by glutamate (Satoh, et al., 2004). By supporting the proposed redox mechanism (FIGS. 5), GSH consumption is reduced and available GSH is recycled and increased (Pawlas, et al., 2007). Therefore, in a condition where GPx activity and GSH level are both in deficit, as in most samples of SZ, ebselen may provide treatment for a GPx mediated disorder due to an impaired GPx/GSH system.

### Example 4: Drug metabolism and pharmacokinetics of ebselen

Ebselen has excellent oral availability (Fisher et al., 1988). Brain level was about 20% of the plasma level (Imai, et al., 2001, Ullrich, et al., 1996). Its neuroprotective effect is observed at 10 uM of plasma level (Zhao, et al., 2002). A Ph-1 study was conducted in 32 humans in a placebo-controlled, randomized, single ascending dose design. Ebselen ranged from 200 mg to1600 mg in the formulation. (1) Pharmacokinetics: The PK parameters of ebselen and its three metabolites were published (Lynch, et al., 2009). Briefly, the mean ebselen Cₘₐₓ ranged from 30.3 ng/mL to 83.4 ng/mL; the mean AUC₀₋ₜ ranged from 117.4 ng*hr/mL to 880.6 ng*hr/mL; the median Tₘₐₓ ranged from 1.5 to 2.3 hours; and the mean t_{1/2} ranged 6.4-16.7 hours. 2-glucuronyl selenobenzanilide was the predominate metabolite. (2) Safety: There were no serious adverse events (AEs) or discontinuation due to an AE. All AEs were mild or moderate and resolved without sequelae. No treatment- or dose-related trends in any clinical laboratory and ECG findings were observed. The tabulated side effects were published in Lynch, et al., 2009. Ebselen capsules in a single oral dose up to 1600 mg appeared to be safe and well tolerated by the healthy males and females.

Seven acute and repeated dose toxicology studies were conducted in Sprague-Dawley rats, Sinclair miniature swine, and Cynomolgus monkey, and three genotoxicology studies. Acute doses of up to 2000 mg/kg were not associated with evidence of acute or delayed toxicity in these species. Repeat dosing 28-day studies in Cynomolgus monkeys established the no-observable-effect-level (NOAEL) to be 2000 mg/kg; over 2000 mg/kg in mini-pig, and NOAEL was not identified for rats. Chronic toxicity studies administering ebselen for 26 and 52 weeks showed that the non-toxic effect level of ebselen was 31.6 mg/kg in rats and 178 mg/kg in mini-pigs.

### Example 5: N-acetyl cysteine treatment in a rat model of psychosis/schizophrenia

This example describes reversal of prepulse inhibition through treatment of ebselen. One of the most replicated findings in schizophrenia research is a reduction of markers associated with cortical inhibitory interneurons (Lewis et al., 2012). Adult neonatal ventral hippocampal lesion (NVHL) rats exhibit electrophysiological anomalies caused by altered cortical interneuron maturation, characterized by abnormal modulation by dopamine (Tseng et al., 2008). Whether parvalbumin (PV) positive interneurons in the PFC, including the dorsal prelimbic and anterior cingulate cortex (ACC), are altered in NVHL rats using unbiased stereological counting techniques was assessed. Between postnatal day (P) 21 and P61, the number of PV immunoreactive interneurons (PVI) increased in sham-operated rats, but not in NVHL rats as illustrated in FIGS. 7A and 1B. In juvenile rats (P21), there was no significant difference in PVI counts between NVHL and sham rats, but adult (P61) NVHL rats showed significantly fewer PVI in the prefrontal cortex (PFC) compared to sham rats. The PVI reduction was prevented with ***N-acetyl cysteine*** (NAC) treatment starting at P5 (i.e., 2 days prior to the hippocampal lesion) and lasting into adolescence (P50; shown in FIGS. 7A-C), suggesting juvenile oxidative stress induced by the neonatal lesion impairs PVI maturation. Caspase 3 labeling did not reveal apoptotic activation in the PFC of NVHL rats (data not shown), suggesting that reduced PVI immunoreactivity more likely reflects reduced interneuron activity than cell loss.

To assess oxidative stress, DNA oxidation with 8-oxo-7, 8-dihydro-20-deoxyguanine (8-oxo-dG) labeling was quantified. At P21, NVHL rats exhibited a massive increase in 8-oxo-dG staining in the PFC compared to sham rats, in both pyramidal neurons and interneurons, which was completely prevented by NAC treatment (as shown in FIGS. 8A and 9B). When NVHL rats reached adulthood (P61), they still showed increased 8-oxo-dG, albeit less than at P21 (as shown in FIGS. 8C and 8D). An increase in 3-Nitrotyrosine (3-NT) levels was observed in adult PFC of NVHL rats. 3-NT indicates nitration of proteins due to oxidative and nitrosative stress (Radi, 2004), and its increase in NVHL rats was prevented by NAC treatment during development (as shown in FIGS. 9A and 9B). Thus, juvenile NAC treatment decreased multiple markers of oxidative stress in adult NVHL rats to levels comparable to control rats, without affecting the extent of the lesion (as shown in FIGS. 10A-10C). A possible explanation for the levels of oxidative stress detected in the adult PFC following an NVHL is the reduced glutamatergic input from ventral hippocampus during development, as blocking NMDA receptors induces oxidative stress in PVI (Behrens et al., 2007). The data indicated that impairing hippocampal inputs to the PFC during a critical developmental period elicits PFC oxidative stress in juvenile rats that has deleterious effects on the adolescent maturation of PVI.

To determine the types of interneurons expressing oxidative stress in NVHL rats, 8-oxo-dG with PV, calbindin (CB) and calretinin (CR) was co-labeled. In addition to pyramidal neurons, increased 8-oxo-dG staining was observed in PVI, but not in CB or CR interneurons (as shown in FIGS. 11A and 11B). About 50% of PVI were co-labeled with 8-oxo-dG, indicating oxidative stress is pervasive in this cell population. A marker of PVI maturation is Wisteria Floribunda agglutinin (WFA), a lectin that recognizes the perineuronal nets (PNN) enwrapping mature cortical PVI. The NVHL lesion reduced WFA staining (as shown in FIGS. 12A-12B), suggesting that PVI in adult PFC of NVHL rats show an immature phenotype. These extracellular matrix alterations were restored with juvenile NAC treatment (as shown in FIGS. 12A-12B). PVI may be highly exposed to increased oxidative stress because they make up the majority of fast-spiking interneurons and their high energy metabolism may generate more reactive oxygen species than non-fast spiking neurons. It is possible that juvenile PVI are functional while exhibiting oxidative stress, with the deleterious effects of oxidative stress becoming evident upon periadolescent PVI maturation.

If juvenile oxidative stress is the cause of physiological anomalies observed in adult NVHL rats, NAC treatment can rescue these alterations. Whole-cell recordings were conducted from pyramidal neurons in adult brain slices containing the medial PFC of SHAM (n=12), NVHL (n=16), and NAC-treated NVHL rats (n=14). As previously shown in adult NVHL rats and other rodent models of schizophrenia (Niwa et al., 2010; Tseng et al., 2008), the dopamine D2-dependent modulation of excitatory postsynaptic potentials (EPSPs) in layer V pyramidal cells was lost in NVHL rats (as shown in FIGS. 13A-C). This loss is likely due to abnormal maturation of PFC interneurons, as the normal adult D2 modulation includes a GABA-A receptor component (Tseng and O'Donnell, 2007), but oxidative stress in pyramidal neurons may also play a role. To determine whether altered PVI-dependent PFC synaptic responses are due to oxidative stress, rats were treated with NAC during development and then tested for D2 modulation of PFC physiology. NAC treatment rescued the D2 modulation of synaptic responses in NVHL rats (as shown in FIGS. 13A-13C), indicating that juvenile and adolescent oxidative stress in NVHL rats alters excitation-inhibition balance in the adult PFC.

The abnormal dopamine modulation of PFC function in NVHL rats is also observed *in vivo. In vivo* intracellular recordings were performed in 38 pyramidal neurons from adult rats (n=9 SHAM (placebo), n=5 NVHL, and n=7 NAC-treated NVHL). Baseline activity was consistent with what has been previously reported for PFC pyramidal neurons (Lewis and O'Donnell, 2000), and was not significantly affected by lesion status or NAC treatment. All recorded cells exhibited spontaneous transitions between the resting membrane potential (down state; -76.2 ± 1.1 mV) and the up state (-67.6 ± 0.7 mV). Up states occurred at a frequency of 0.6 ± 0.1 Hz with a duration of 523.6 ± 24.7 ms. The majority of cells (n=21) fired spontaneously at a rate of 2.1 ± 0.7 Hz. As previously reported (O'Donnell et al., 2002), *in vivo* intracellular recordings from anesthetized adult NVHL rats revealed an abnormal increase in pyramidal cell firing in response to burst stimulation of the Ventral Tegmental Area (VTA) (as shown in FIGS. 13D and 13E) compared to sham rats. This abnormal increase in firing was prevented by juvenile NAC treatment (as shown in FIGS. 13D and 13E). These data indicate abnormal dopamine function in the PFC of NVHL rats depends on oxidative stress during juvenile and adolescent stages.

Abnormal excitation-inhibition balance in adult NVHL rats can yield altered information processing that would be prevented by NAC treatment if it depended on oxidative stress. Mismatch negativity (MMN) was tested using auditory evoked potentials in an oddball paradigm in SHAM (n=6), NVHL (n=3), and NAC-treated NVHL rats (n=3). MMN has high translational relevance, as it is attenuated in schizophrenia patients (Javitt et al., 1993) and in animal models (Ehrlichman et al., 2009). Electroencephalographic (EEG) electrodes were implanted in NVHL, NAC-treated NVHL, and SHAM rats. MMN was significantly different among groups, with NAC treatment improving MMN in NVHL rats (as shown in FIGS. 14A and 14B). This observation is consistent with the effect of NAC on MMN in patients (Lavoie et al., 2008), and indicates the NVHL model reproduces an important disease marker that can be prevented by juvenile antioxidant treatment. As MMN depends on NMDA receptor function (Umbricht et al., 2000) and NMDA hypofunction in PVI is suspected in schizophrenia, it is possible that MMN improvement with NAC results from restored PVI activity.

To assess whether juvenile oxidative stress leads to behavioral deficits, a behavioral paradigm was used for testing in both animal models and schizophrenia patients. Prepulse inhibition of the acoustic startle response (PPI) is a measure of sensorimotor gating that is reduced in patients (Geyer and Braff, 1987) and NVHL rats (Lipska et al., 1995). PPI was tested in adult sham (n=11), NAC-treated sham (n=12), NVHL (n=9), and NAC- treated NVHL rats (n=17). Juvenile NAC treatment prevented the reduced PPI observed in untreated NVHL rats (as shown in FIG. 15A). In addition to loss of PVI maturation and electrophysiological anomalies, developmental oxidative stress in juvenile NVHL rats can cause schizophrenia-relevant adult behavioral deficits.

The beneficial effect of NAC treatment includes a large postnatal treatment that starts prior to the lesion and stops once rats become young adults. For full translational value one determines whether NAC is efficacious when started at an age that corresponds to the time when prodromal stages can be identified in humans. In another set of rats, NAC was administered in the drinking water starting at P35, an age that in rats is equivalent to early adolescence. For PPI deficits was tested in adult SHAM (n=15), untreated NVHL (n=12), and NAC-treated NVHL rats (n=14). Showing a trend for a deficit in untreated NVHL rats compared to shams in this group, there was a significant difference between untreated and treated NVHL (as shown in FIG. 15B). The data indicate that GSH precursors such as NAC can still be effective even if initiated after oxidative stress has begun.

One important caveat of NAC is that it also alters glutamate levels by virtue of its action on the cysteine-glutamate transporter (Moussawi et al., 2009). To test whether redox modulation and not glutamate level changes were responsible for NAC effects in NVHL rats, the effect of two other antioxidants was assessed that do not alter glutamate.

### Example 6: Ebselen/Apocynin treatment in a rat model of psychosis/schizophrenia

***Ebselen*** is a glutathione peroxidase (GPx) mimic (Muller et al., 1984) that induces GPx expression (Kil et al., 2007) and enhances GSH levels in neurons, replenishing GSH depleted by neurotoxic mechanisms (Pawlas and Malecki, 2007). PPI was tested in adult vehicle-treated SHAM (n=10), Ebselen-treated SHAM (n=7), vehicle-treated NVHL (n=8), and Ebselen-treated NVHL rats (n=9), and Ebselen treatment during adolescence reversed PPI deficits in NVHL rats (as shown in FIG. 15D).

In another group of rats, the effects of the NADPH oxidase inhibitor ***Apocynin*** was assessed while delivering through juvenile and adolescent stages. PPI was tested in adult vehicle-treated SHAM (n=10), apocynin-treated SHAM (n=11), vehicle treated NHVL (n=7), and apocynin-treated NVHL (n=5). As illustrated in FIG. 15C a reversal of PPI deficits was observed. The data indicated that elevation of GSH and not glutamate during adolescence rescues PPI deficits in NVHL rats.

### Experimental Procedures

Animals: Timed-pregnant Sprague-Dawley rats were obtained at gestational days 13-15 from Charles River (Wilmington, MA) and were individually housed with free access to food and water in a temperature- and humidity-controlled environment with a 12:12h light/dark cycle (lights on at 7:00 AM). When pups reached P5, half of the dams received NAC in their drinking water. Pups were left undisturbed until P7-9 when healthy offspring were randomly separated and received either NVHL or sham surgery. At P21, male and female pups were either transcardially perfused with 4% paraformaldehyde for immunocytochemistry or weaned and housed in groups of two to three, counterbalanced across lesion status. NAC treatment lasted throughout adolescence until P50. After reaching adulthood (>P60), animals were either perfused with 4% paraformaldehyde for immunocytochemistry, perfused with artificial cerebrospinal fluid (aCSF) for slice electrophysiology, utilized for *in vivo* intracellular recordings, or tested for PPI or MMN.

Neonatal ventral hippocampal lesion surgery: Between P7 and P9, pups (15-20 g) received either an excitotoxic lesion of the ventral hippocampus (NVHL) or sham procedure, as previously described (Chambers and Lipska, 2011). Pups were anesthetized with hypothermia and secured to a Styrofoam platform attached to a stereotaxic frame (David Kopf Instruments, Tujunga, CA). NVHL rats received a bilateral infusion of ibotenic acid (10 µg/µl in aCSF, 0.3 µl/side; Tocris, Minneapolis, MN) into the ventral hippocampus (3 mm rostral to Bregma, 3.5 mm lateral to midline, and 5 mm from surface) at a rate of 0.15 µl/min. Sham surgeries were done in exactly the same fashion, but the guide cannula was lowered only 3 mm and without any liquid infusion to control for the surgical procedure while avoiding hippocampal damage. After the surgery, wounds were clipped and when pups activity level had returned to normal, they were returned to their dams and remained undisturbed until the wound clips were removed and rats weaned at P21.

In all rats, lesions were verified by sectioning (40 µm) the dorsal and ventral hippocampus using a freezing microtome. Sections were mounted on glass slides and Nissl stained. The hippocampus was examined microscopically for evidence of bilateral damage, which typically included cell loss, thinning, gliosis, cellular disorganization and enlarged ventricles (Chambers and Lipska, 2011).

Antioxidant pretreatment regimen: NAC (BioAdvantexPharma, Mississauga, Ontario, Canada) was administered in the drinking water at 900 mg/l. NAC treatment started at P5 or at P35, and previous work in mice has shown that NAC consumed by the dam is transmitted to the pups through her milk (das Neves Duarte et al., 2012). NAC treatment ended at P50. Fresh solutions were prepared every 2-3 days. Ebselen (Sound Pharmaceuticals Inc., Seattle, WA) was administered i.p. 5 days a week starting at P35 until the day of PPI testing (P60). Stock ebselen solution (20 mg/ml DMSO, frozen aliquots) was diluted 1:5 in sterile water and administered at a dose of 10 mg/kg. Control animals received an equivalent concentration of DMSO diluted 1:5 in water. Apocynin (Sigma-Aldrich, St. Louis, MO) was administered in the drinking water at a target dose of 100 mg/kg (Nwokocha et al., 2013). Prior to weaning at P21, drinking water contained a dose of 2 g apocynin per 0.51 of water, to ensure delivery through the dam's milk. Apocynin concentration was lowered after weaning to 750 mg/l to best approximate the target dose. Treatment lasted from P5 to P50 with fresh solutions prepared every other day.

Immunohistochemistry and stereological quantification: A total of 18 (P21) and 25 (P61) male rats were anesthetized, perfused and their brains fixed as previously described (Cabungcal et al., 2006). Coronal sections (40 µm) were used to investigate the inhibitory circuitry of anterior cingulate cortex (ACC). Brain sections were immunolabeled for parvalbumin (PV) as described previously (Steullet et al., 2010). PV-immunoreactive cell (cell bodies) count was quantified in ACC using the StereoInvestigator 7.5 software (MBF Bioscience Inc, Williston, VT, USA). Briefly, stereological counting started with low magnification (x2.5 objective) to identify and delineate the boundaries of the region of interest (ROI) on 2-4 consecutive sections from each animal. The ACC (at Bregma approximately 0.70-1.70 mm) was delineated from the secondary motor (M2) cortical regions following the anatomical cytoarchitectonic areas given by Paxinos and Watson (Paxinos and Watson, 1998). The selected region of interest (ROI) included the majority of the cingulate cortex area 1 (cg1) and part of cingulate cortex area 2 (cg2). A small intermediate allowance was set between ACC and M2 regions to ensure that the ROI in ACC did not overlap with the secondary motor cortex. A counting box (optical dissector) within the section thickness and sampling frames adapted to ACC were used to analyze and count neurons (Schmitz and Hof, 2005). The counting boxes (40 x 40 µm with 15 µm in depth) were placed by the software in each sampling frame starting from a random position inside the ROI of the ACC. Counting was carried out using higher magnification (x40 objective). PV cells were counted when they were in focus at the surface of the box until out of focus at 15-µm depth of the counting box. A 5-µm guard zone was used to distance from artifacts that can be influenced by tissue shrinkage due to the immunopreparation processing. 25 counting frames were used in the ROI volume of the ACC for P21 and P61 rats.

Immunofluorescence staining, confocal microscopy and image analysis: Oxidative stress was visualized using an antibody against 8-oxo-7, 8-dihydro-20- deoxyguanine (8-Oxo-dG), a DNA adduct formed by the reaction of OH radicals with the DNA guanine base (Kasai, 1997). Because of the proximity of the electron transport chain, mitochondrial DNA is prone to oxidative damage: levels of oxidized bases in DNA and levels of 8-oxo-dG are higher in mitochondria than in the nucleus. To assess 8-oxo- dG and 3-Nitrotyrosine (3NT) labeling in various types of interneurons, coronal sections between Bregma 0.70-1.70 mm were incubated for about 36 hours with rabbit polyclonal anti-PV, anti-calbindin-28k (anti-CB), or anti-calretinin (anti-CR) (1:2500; Swant, Bellinzona, Switzerland) primary antibodies together with the mouse monoclonal anti-8-oxo-dG (1:350; AMS Biotechnology, Bioggio-Lugano, Switzerland) primary antibody or mouse monoclonal anti-nitrotyrosine (1: 1000; Chemicon International, Temecula, USA) primary antibody. To enable visualization of the PNN that surrounds PV cells, sections were incubated in a solution containing the biotin-conjugated lectin *Wisteria floribunda agglutinin* (WFA) (Hartig et al., 1994). Sections were first incubated with PBS + Triton 0.3% + sodium azide (1 g/l) containing 2% normal horse serum, followed by 36-hour incubation with rabbit polyclonal anti-PV (1:2500) and biotin conjugated-WFA (1:2000; Sigma). Sections were washed, incubated with appropriate fluorescent secondary antibodies (goat anti-mouse immunoglobulin G (1:300; Alexa Fluor 488; Molecular Probes, Eugene, Oregon), anti-rabbit immunoglobulin G (1:300; CY3; Chemicon International, Temecula, California), CY2-Streptavidin conjugate (1:300; Chemicon), and counterstained with 100 ng/ml DAPI (4'-6-diamidino-2-phenylindole; Vector Laboratories, California, USA). Sections were visualized with a Zeiss Confocal Microscope equipped with x10, x20, x40 and x63 Plan-NEOFLUAR objectives. All peripherals were controlled with LSM 510 software (Carl Zeiss AG, Switzerland). Z stacks of 9 images (with a 2.13 µm interval) were scanned (1024 x 1024 pixels) for analysis in IMARIS 7.3 software (Bitplane AG, Switzerland). All images of Z stacks were filtered with a Gaussian filter tool to remove unwanted background noise and sharpen cell body contours. An ROI as defined in the stereological procedure was created in ACC. The ROI was masked throughout the Z stacks to isolate regional subvolumes of the ACC in which PV-, CB-, and CR-expressing interneurons were analyzed. To quantify 8-oxo-dG, the staining intensity and number of labelled voxels within the ROI were measured. To quantify 8-oxo-dG in PV-, CB- and CR-cells, we used the *Coloc* module of the IMARIS software to calculate the proportion of all PV-immunolabeled voxels (respectively, CB- and CR-immunolabeled voxels), which were also 8-oxo-dG- immnolabeled. *Coloc* gives the count of colocalized voxels between the immunolabeled profiles of interest. To quantify the number of PV immunoreactive neurons surrounded by PNN, we used the spots module to assign spot markings on profile-labelled voxels that fall within a given size. The channels for PV and WFA immunolabeling were chosen, and profile size criterion (>9 and 4 µm, respectively) was defined to quantify labelled profiles above these sizes. Spots generated for PV that contacted and/or overlapped with spots generated for WFA were considered as those PVI surrounded by PNN (WFA-positive PVI).

Slice electrophysiology: Starting at P60, male rats were anesthetized with chloral hydrate (400 mg/kg, i.p.) 15 min before being decapitated. Brains were quickly removed from the skull into ice-cold artificial CSF (aSCF) oxygenated with 95% O₂-5% CO₂ and containing the following (in mM): 125 NaCl, 25 NaHCO₃, 10 glucose, 3.5 KCl, 1.25 NaH₂PO₄, 0.5 CaCl₂ and 3 MgCl₂, pH 7.45 (295-300 mOsm). Coronal slices (300 µm thick) containing the medial PFC were obtained with a vibratome in ice-cold aCSF and incubated in warm (~35° Celsius) aCSF solution constantly oxygenated with 95% O₂-5% CO₂ for at least 45 min before recording. The recording aCSF (with 1 CaCl₂ and 2 MgCl₂) was delivered to the recording chamber with a pump at the rate of 2 ml/min.

Patch electrodes (7-10 MΩ) were obtained from 1.5 mm borosilicate glass capillaries (World Precision Instruments) with a Flaming-Brown horizontal puller (P97; Sutter Instruments) and filled with a solution containing 0.125% Neurobiotin and the following (in mM): 115 K-gluconate, 10 HEPES, 2 MgCl₂, 20 KCl, 2 MgATP, 2 Na₂-ATP, and 0.3 GTP, pH 7.25-7.30 (280-285 mOsm). Quinpirole (5 µM, Tocris) was freshly mixed into oxygenated recording aCSF every day before an experiment. Both control and drug-containing aCSF were oxygenated continuously throughout the experiments.

All experiments were conducted at 33-35° Celsius and prelimbic or ACC PFC pyramidal cells from layer V were identified under visual guidance using infrared (IR) differential interference contrast video microscopy with a 40X water-immersion objective (Olympus BX-51WI). The image was detected with an IR-sensitive CCD camera and displayed on a monitor. Whole-cell current-clamp recordings were performed with a computer- controlled amplifier (Multiclamp 700A; Molecular Devices), digitized (Digidata 1322; Molecular Devices), and acquired with Axoscope 9 (Molecular Devices) at a sampling rate of 10 kHz. Electrode potentials were adjusted to zero before recording without correcting the liquid junction potential. Baseline activity in each neuron was monitored for 10 minutes during which membrane potential and input resistance (measured with the slope of a current-voltage (I/V) plot obtained with 500-ms-duration depolarizing and hyperpolarizing pulses) were measured.

Synaptic responses were tested in pyramidal neurons with electrical stimulation of superficial layers with a bipolar electrode made from a pair of twisted Teflon-coated Tungsten wires (tips separated by ~200 µm) and placed ~500 µm lateral to the vertical axis of the apical dendrite of the recorded neuron. Stimulation pulses (20-400 µA; 0.5 ms) were delivered every 15 seconds. The intensity was adjusted to evoke EPSPs with about half of the maximal amplitude. Throughout the experiment, changes in input resistance were monitored with repeated hyperpolarizing steps, and the cell was discarded when input resistance changed more than 20% during the course of the experiment. The amplitude of evoked EPSPs was measured with Clampfit 9.0 and averaged over 10 sweeps before and after 7 minutes of application of quinpirole. This period was chosen for consistency, with differences revealed by previous investigations of D2 modulation of PFC activity in rodent models of schizophrenia (Niwa et al., 2010; Tseng et al., 2008). At the end of each experiment, slices were placed in 4% paraformaldehyde and processed for DAB staining using standard histochemical techniques to verify morphology and location of the neurons.

In Vivo intracellular recordings: Female rats were anesthetized with choral hydrate (400 mg/kg, i.p) and placed on a stereotaxic apparatus (Kopf Instruments). Anesthesia was maintained through recording procedures with continuous choral hydrate (24-30 mg/kg/h) via an intraperitoneal catheter. Body temperature was maintained at approximately 37° Celsius using a thermal probe-controlled heat pad (Fine Science Tools). Concentric bipolar stimulating electrodes (0.5 mm diameter, 0.5 mm pole separation; Rhodes Medical Instruments Inc.) were lowered into the VTA (5.8 mm caudal to bregma; 0.5-0.8 mm lateral to midline; 7-8 mm from surface) for stimulation. Recording sharp micro-electrodes were pulled from borosilicate glass (1 mm O.D.; World Precision Instruments) on a horizontal Flaming-Brown puller (Sutter Instruments). Sharp electrodes (50-110 MΩ) were filled with 2% Neurobiotin (Vector Laboratories) in 2M potassium acetate. Microelectrodes were lowered into the medial PFC using a hydraulic manipulator (Trent Wells, Coulterville, CA). Recordings were made in current clamp, and signals were acquired using a Neurodata Amplifier (Cygnus), digitized at 10 kHz using a Digidata A/D converter (Molecular Devices) and Axoscope 9 software (Molecular Devices) for offline analyses.

Microelectrodes were advanced through the medial PFC until a neuron was impaled. Neurons included in this study had a resting membrane potential more negative than - 60 mV and action potentials with amplitudes ≥ 40 mV from threshold. To determine responses to endogenous dopamine, the VTA was stimulated with trains of 5 pulses at 20 Hz, delivered every 10 seconds. Eight to ten sweeps were used to determine cell firing in response to VTA stimulation. Firing was measured in the 500 ms epoch following the last VTA pulse in all sweeps, and compared among experimental groups. At the end of the experiment, animals were killed with anesthesia overdose, and their brains removed for histological verification of lesion status and electrode placement.

Mismatch Negativity: NVHL, NAC-treated NVHL, and sham female rats were implanted with chronic EEG electrodes under isoflurane anesthesia. Electrodes were constructed with 2 mm diameter silver disks coated with silver chloride, and glued on top of bregma, a location equivalent to human vertex, and the contacts led to an *Omnetics* connector on top of the head. Upon a 4-week recovery, rats were first habituated to the recording chamber, a 30 x 50 cm plexiglass box enclosed within a stainless steel box. NNM sessions consisted of exposing the rat to approximately 2,000 tones at two different frequencies (7 or 9 kHz; 30 ms duration) separated by 400 ms, with 95% of the repetitions at one frequency (standard) and 5% at the other frequency (deviant). Tones were delivered with a speaker mounted inside the enclosure using a TDT RZ6 system (Tucker Davis), and were counterbalanced so half of the time the deviant was either frequency. EEG signals were acquired using a 32 channel Omniplex system (Plexon Instruments) at 1 kHz sampling rate. For analysis, 300 ms epochs around the tone were selected, filtered at 1-30 Hz, baseline-corrected to the 100 ms prior to the stimulus, and averaged separately for standard and deviant tones. A difference wave was constructed by subtracting the standard wave from the deviant wave, and MMN was quantified by measuring the area under the curve in the period between 35 and 100 ms after the stimulus. All rats were exposed to three sessions in three different days, and values were averaged across sessions for every animal.

Prepulse inhibition: Starting at P60, both male and female rats were tested for PPI, as described previously (Feleder et al., 2010). As PPI deficits in NVHL rats are most evident when rats are challenged with apomorphine (Lipska et al., 1995), we injected apomorphine (0.1 mg/kg, i.p.) immediately prior to the PPI test session. Rats were placed in a sound- attenuated startle chamber (San Diego Instruments, San Diego, CA) with a 70 dB background white noise. After a 5 min adaptation period, the PPI test was initiated with pseudorandom trials every 15 to 25 seconds. Either pulse (120 dB), prepulse (75 dB, 80 dB, or 85 dB), no pulse or prepulse + pulse were delivered. Trials lasted 23 min and 8 to 10 repetitions of pulse or prepulse + pulse trials were acquired, while null or prepulse only trials were repeated five times for each prepulse amplitude. Startle magnitude was measured using an acceleration-sensitive transducer, and PPI was calculated as the ratio in startle between prepulse + pulse and pulse alone and is expressed as percent reduction. The initial trials (all pulse alone) were used for habituation and not included in the analysis. Trials were excluded from analysis when the animal was moving in the chamber, and sessions were excluded from analysis when startle amplitude was low or more than 50% of trials were excluded for any prepulse + pulse combination. If a PPI session was discarded, rats were tested again a week later.

Statistics: The mean numbers of PV-immunoreactive cells per tissue volume in the ACC were compared among treatment groups using one-way ANOVA followed by post-doc Dunnett multiple comparisons. The mean number of PV-cells, PV-cell intensity, WFA-positive PV and WFA-positive intensity, the overall 8-oxo-dG, and WFA labelling were compared among groups using multivariate ANOVA (Wilk's Lambda) followed by post- hoc Dunnett test for multiple comparisons. Electrophysiology data were compared using a 1-way ANOVA with group as between-subject variable. PPI data were compared using a repeated-measures 2-way ANOVA with lesion status and treatment as between- subject variables, and prepulse intensity as within-subject variable.

## Claims

1. A glutathione peroxidase modulator compound and antipsychotic agent for use in a method of treating a psychotic disorder, wherein the psychotic disorder is bi-polar disorder or schizophrenia, wherein the antipsychotic agent is co-administered with the glutathione peroxidase modulator compound, and wherein an effective dosage of the antipsychotic agent in the presence of the co-administered glutathione peroxidase modulator compound is lower than an effective dose of the antipsychotic compound in the absence of the glutathione peroxidase modulator compound, wherein the glutathione peroxidase modulator compound is ebselen.

2. The glutathione peroxidase modulator compound and antipsychotic agent for use of claim 1, wherein the co-administration reduces a side effect of administering the antipsychotic agent.

3. The glutathione peroxidase modulator compound and antipsychotic agent for use of claim 1 or 2, wherein the antipsychotic agent is selected from a group consisting of:
Chlorpromazine (Thorazine), Haloperidol (Haldol), Perphenazine, Fluphenazine, Risperidone (Risperdal), Olanzapine (Zyprexa), Quetiapine (Seroquel), Ziprasidone (Geodon), Aripiprazole (Abilify), Paliperidone (Invega), Lurasidone (Latuda) and combinations thereof.

4. The glutathione peroxidase modulator compound and antipsychotic agent for use of claim 1, wherein the psychotic disorder is bi-polar disorder.

5. The glutathione peroxidase modulator compound and antipsychotic agent for use of claim 1, wherein the psychotic disorder is schizophrenia.

6. The glutathione peroxidase modulator compound and antipsychotic agent for use of claim 3, wherein the antipsychotic agent is Risperidone.

7. The glutathione peroxidase modulator compound and antipsychotic agent for use of claim 3, wherein the antipsychotic agent is Aripiprazole.

8. The glutathione peroxidase modulator compound and antipsychotic agent for use of claim 2, wherein the side effect is tardive dyskinesia.

9. The glutathione peroxidase modulator compound and antipsychotic agent for use of claim 3-5, wherein the antipsychotic agent is Quetiapine.

10. A pharmaceutical composition comprising a combination of therapeutic agents, said combination consisting of:
(i) ebselen or a pharmaceutically acceptable salt thereof; and
(ii) an antipsychotic agent or a pharmaceutically acceptable salt thereof.

11. The pharmaceutical composition of claim 10, wherein the antipsychotic agent is selected from a group consisting of: Chlorpromazine (Thorazine), Haloperidol (Haldol), Perphenazine, Fluphenazine, Risperidone (Risperdal), Olanzapine (Zyprexa), Quetiapine (Seroquel), Ziprasidone (Geodon), Aripiprazole (Abilify), Paliperidone (Invega), Lurasidone (Latuda) and combinations thereof.

12. The pharmaceutical composition of claim 11, wherein the antipsychotic agent is Risperidone.

13. The pharmaceutical composition of claim 11, wherein the antipsychotic agent is Aripiprazole.

14. The pharmaceutical composition of claim 11, wherein the antipsychotic agent is Quetiapine.

## Patentansprüche

1. Glutathionperoxidase-Modulatorverbindung und Antipsychotikum zur Verwendung bei einem Verfahren zum Behandeln einer psychotischen Erkrankung, wobei die psychotische Erkrankung bipolare Erkrankung oder Schizophrenie ist, wobei das Antipsychotikum zusammen mit der Glutathionperoxidase-Modulatorverbindung verabreicht wird und wobei eine wirksame Dosierung des Antipsychotikums in der Gegenwart der gleichzeitig verabreichten Glutathionperoxidase-Modulatorverbindung niedriger ist als eine wirksame Dosis der Antipsychotikumverbindung in der Abwesenheit der Glutathionperoxidase-Modulatorverbindung, wobei die Glutathionperoxidase-Modulatorverbindung Ebselen ist.

2. Glutathionperoxidase-Modulatorverbindung und Antipsychotikum zur Verwendung nach Anspruch 1, wobei die gleichzeitige Verabreichung eine Nebenwirkung einer Verabreichung des Antipsychotikums reduziert.

3. Glutathionperoxidase-Modulatorverbindung und Antipsychotikum zur Verwendung nach Anspruch 1 oder 2, wobei das Antipsychotikum ausgewählt ist aus einer Gruppe bestehend aus: Chlorpromazin (Thorazin), Haloperidol (Haldol), Perphenazin, Fluphenazin, Risperidon (Risperdal), Olanzapin (Zyprexa), Quetiapin (Seroquel), Ziprasidon (Geodon), Aripiprazol (Abilify), Paliperidon (Invega), Lurasidon (Latuda) und Kombinationen davon.

4. Glutathionperoxidase-Modulatorverbindung und Antipsychotikum zur Verwendung nach Anspruch 1, wobei die psychotische Erkrankung bipolare Erkrankung ist.

5. Glutathionperoxidase-Modulatorverbindung und Antipsychotikum zur Verwendung nach Anspruch 1, wobei die psychotische Erkrankung Schizophrenie ist.

6. Glutathionperoxidase-Modulatorverbindung und Antipsychotikum zur Verwendung nach Anspruch 3, wobei das Antipsychotikum Risperidon ist.

7. Glutathionperoxidase-Modulatorverbindung und Antipsychotikum zur Verwendung nach Anspruch 3, wobei das Antipsychotikum Aripiprazol ist.

8. Glutathionperoxidase-Modulatorverbindung und Antipsychotikum zur Verwendung nach Anspruch 2, wobei die Nebenwirkung tardive Dyskinesie ist.

9. Glutathionperoxidase-Modulatorverbindung und Antipsychotikum zur Verwendung nach Anspruch 3-5, wobei das Antipsychotikum Quetiapin ist.

10. Pharmazeutische Zusammensetzung, umfassend eine Kombination von therapeutischen Mitteln, wobei die Kombination aus Folgendem besteht:
(i) Ebselen oder einem pharmazeutisch unbedenklichen Salz davon; und
(ii) einem Antipsychotikum oder einem pharmazeutisch unbedenklichen Salz davon.

11. Pharmazeutische Zusammensetzung nach Anspruch 10, wobei das Antipsychotikum ausgewählt ist aus einer Gruppe bestehend aus: Chlorpromazin (Thorazin), Haloperidol (Haldol), Perphenazin, Fluphenazin, Risperidon (Risperdal), Olanzapin (Zyprexa), Quetiapin (Seroquel), Ziprasidon (Geodon), Aripiprazol (Abilify), Paliperidon (Invega), Lurasidon (Latuda) und Kombinationen davon.

12. Pharmazeutische Zusammensetzung nach Anspruch 11, wobei das Antipsychotikum Risperidon ist.

13. Pharmazeutische Zusammensetzung nach Anspruch 11, wobei das Antipsychotikum Aripiprazol ist.

14. Pharmazeutische Zusammensetzung nach Anspruch 11, wobei das Antipsychotikum Quetiapin ist.

## Revendications

1. Composé modulateur de la glutathion peroxydase et agent antipsychotique destinés à être utilisés dans un procédé de traitement d'un trouble psychotique, ledit trouble psychotique étant un trouble bipolaire ou la schizophrénie, ledit agent antipsychotique étant co-administré avec le composé modulateur de la glutathion peroxydase, et une dose efficace de l'agent antipsychotique en présence du composé modulateur de la glutathion peroxydase co-administré étant inférieure à une dose efficace du composé antipsychotique en l'absence du composé modulateur de la glutathion peroxydase, ledit composé modulateur de la glutathion peroxydase étant l'ebselen.

2. Composé modulateur de la glutathion peroxydase et agent antipsychotique destinés à être utilisés selon la revendication 1, ladite co-administration réduisant un effet secondaire de l'administration de l'agent antipsychotique.

3. Composé modulateur de la glutathion peroxydase et agent antipsychotique destinés à être utilisés selon la revendication 1 ou 2, ledit agent antipsychotique étant choisi dans un groupe constitué de : la chlorpromazine (Thorazine), l'halopéridol (Haldol), la perphénazine, la fluphénazine, la rispéridone (Risperdal), l'olanzapine (Zyprexa), la quétiapine (Seroquel), la ziprasidone (Geodon), l'aripiprazole (Abilify), la palipéridone (Invega), la lurasidone (Latuda) et des combinaisons de ceux-ci.

4. Composé modulateur de la glutathion peroxydase et agent antipsychotique destinés à être utilisés selon la revendication 1, ledit trouble psychotique étant un trouble bipolaire.

5. Composé modulateur de la glutathion peroxydase et agent antipsychotique destinés à être utilisés selon la revendication 1, dans lequel le trouble psychotique est la schizophrénie.

6. Composé modulateur de la glutathion peroxydase et agent antipsychotique destinés à être utilisés selon la revendication 3, ledit agent antipsychotique étant la rispéridone.

7. Composé modulateur de la glutathion peroxydase et agent antipsychotique destinés à être utilisés selon la revendication 3, ledit agent antipsychotique est l'aripiprazole.

8. Composé modulateur de la glutathion peroxydase et agent antipsychotique destinés à être utilisés selon la revendication 2, ledit effet secondaire étant une dyskinésie tardive.

9. Composé modulateur de la glutathion peroxydase et agent antipsychotique destinés à être utilisés selon les revendications 3 à 5, ledit agent antipsychotique étant la quétiapine.

10. Composition pharmaceutique comprenant une combinaison d'agents thérapeutiques, ladite combinaison étant constituée :
(i) de l'ebselen ou d'un sel acceptable pharmaceutiquement de celui-ci ; et
(ii) d'un agent antipsychotique ou d'un sel acceptable pharmaceutiquement de celui-ci.

11. Composition pharmaceutique selon la revendication 10, dans laquelle l'agent antipsychotique est choisi dans un groupe constitué de : la chlorpromazine (Thorazine), l'halopéridol (Haldol), la perphénazine, la fluphénazine, la rispéridone (Risperdal), l'olanzapine (Zyprexa), la quétiapine (Seroquel), la ziprasidone (Geodon), l'aripiprazole (Abilify), la palipéridone (Invega), la lurasidone (Latuda) et des combinaisons de ceux-ci.

12. Composition pharmaceutique selon la revendication 11, dans laquelle l'agent antipsychotique est la rispéridone.

13. Composition pharmaceutique selon la revendication 11, dans laquelle l'agent antipsychotique est l'aripiprazole.

14. Composition pharmaceutique selon la revendication 11, dans laquelle l'agent antipsychotique est la quétiapine.
